# EUROPÄISCHE PATENTSCHRIFT

(11) **EP 1 140 907 B1**
(45) Veröffentlichungstag und Bekanntmachung des Hinweises auf die Patenterteilung: **11.07.2007**
(21) Anmeldenummer: 00901535.5
(22) Anmeldetag: 12.01.2000
(51) Int. Cl.: C07D 403/06, C07D 403/14, A61K 31/41, A61P 25/16, A61P 25/18, A61P 25/24, C07D 403/12

(54) **TRIAZOLVERBINDUNGEN MIT DOPAMIN-D3-REZEPTORAFFINITÄT**
TRIAZOLE COMPOUNDS WITH DOPAMINE-D3-RECEPTOR AFFINITY
COMPOSES DE TRIAZOL PRESENTANT UNE AFFINITE POUR LE RECEPTEUR 3 DE LA DOPAMINE

(30) Priorität: 12.01.1999 DE 19900811
(43) Veröffentlichungstag der Anmeldung: 10.10.2001
(73) Patentinhaber: Abbott GmbH & Co. KG, 65205 Wiesbaden (DE)
(72) Erfinder: STARCK, Dorothea, D-67059 Ludwigshafen (DE); TREIBER, Hans-Jörg, D-68782 Brühl (DE); UNGER, Liliane, D-67065 Ludwigshafen (DE); NEUMANN-SCHULTZ, Barbara, D-68526 Ladenburg (DE); BLUMBACH, Kai, D-97337 Dettelbach (DE); SCHÖBEL, Dietmar, D-68167 Mannheim (DE)
(74) Vertreter: Kinzebach, Werner
(86) Internationale Anmeldenummer: PCT/EP2000/000176
(87) Internationale Veröffentlichungsnummer: WO 2000/042037

(56) Entgegenhaltungen:
- EP-A- 0 887 350
- WO-A-96/02520
- WO-A-96/31512
- WO-A-97/17326
- WO-A-97/25324
- WO-A-99/02503
- DE-A- 4 425 144
- US-A- 4 338 453
- US-A- 4 577 020
- A. CZARNOCKA-JANOWICZ ET AL.: "Synthesis and pharmacological activity of 5-substituted-s-triazole-3-thiols" PHARMAZIE, Bd. 46, Nr. 2, 1991, Seiten 109-112, XP002082580 in der Anmeldung erwähnt

## Beschreibung

Die Erfindung betrifft Triazolverbindungen und die Verwendung dieser Verbindungen. Diese besitzen wertvolle therapeutische Eigenschaften und sind zur Behandlung von Erkrankungen brauchbar, die auf die Beeinflussung durch Dopamin-D₃-Rezeptor-Liganden ansprechen.

Verbindungen der hier in Rede stehenden Art mit physiologischer Aktivität sind bereits bekannt. So beschreiben die WO 94/25013; 96/02520; 97/43262; 97/47602; 98/06699; 98/49145; 98/50363; 98/50364 und 98/51671 Verbindungen, welche auf die Dopamin-Rezeptoren wirken. Weitere Verbindungen mit Aktivität als Dopamin-D₃-Rezeptor-Liganden sind aus der DE 44 25 144 A, WO 96/30333 und der WO 97/25324, WO 97/40015, WO 97/47602, WO 97/17326, EP 887 350, EP 779 284 A und aus Bioorg. & Med. Chem. Letters 9 (1999) 2059-2064 bekannt. Triazolverbindungen mit anti-allergischer oder anti-psychotischer Aktivität sind aus US 4,338,453; 4,408,049 und 4,577,020 bekannt. Die WO 93/08799 und WO 94/25013 beschreiben Verbindungen der hier in Rede stehenden Art, die Endothelin-Rezeptorantagonisten darstellen. In Pharmazie 46 (1991), 109-112, sind weitere Triazolverbindungen beschrieben, welche die Blutplättchenaggregation hemmen und blutdrucksenkend wirken. Weitere Triazolverbindungen mit physiologischer Aktivität sind aus EP 691 342, EP 556 119, WO 97/10210, WO 98/24791, WO 96/31512 und WO 92/20655 bekannt.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Störungen im dopaminergen Transmittersystem resultieren in Erkrankungen, wie z.B. Schizophrenie, Depression und Parkinson'sche Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁- und D₂-Rezeptoren.

In jüngerer Zeit wurde ein dritter Subtyp gefunden, nämlich der D₃-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514).

Da D₃-Rezeptoren hauptsächlich im limbischen System exprimiert werden, wird angenommen, dass ein selektiver D₃-Ligand wohl die Eigenschaften bekannter Antipsychotika, nicht aber ihre Dopamin-D₂-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Überraschenderweise wurde nun gefunden, dass bestimmte Triazolverbindungen eine hohe Affinität zum Dopamin-D₃-Rezeptor und eine geringe Affinität zum D₂-Rezeptor aufweisen. Es handelt sich somit um selektive D₃-Liganden.

Gegenstand der vorliegenden Erfindung sind daher die Verbindungen der allgemeinen Formel I: 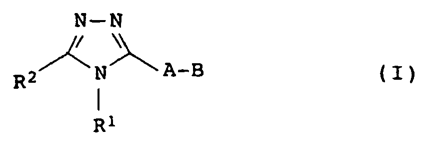 worin
- R¹: für H, C₁-C₆-Alkyl, das gegebenenfalls durch OH, OC₁-C₆-Alkyl, Halogen oder Phenyl substituiert ist, C₃-C₆-Cycloalkyl oder Phenyl steht;
- R²: für H, C₁-C₆-Alkyl, das gegebenenfalls durch OH, OC₁-C₆-Alkyl, Halogen oder Phenyl substituiert ist, C₁-C₆-Alkoxy, C₁-C₆-Alkylthio, C₂-C₆-Alkenyl, C₂-C₆-Alkinyl, C₃-C₆-Cycloalkyl, Halogen, CN, COOR³, CONR³R⁴, NR³R⁴, SO₂R³, SO₂NR³R⁴ oder einen aromatischen Rest steht, der ausgewählt ist unter Phenyl, Naphthyl und einem 5- oder 6-gliedrigen heterocyclischen Rest mit 1, 2, 3 oder 4 Heteroatomen, die unabhängig voneinander ausgewählt sind unter 0, N und S, wobei der aromatische Rest einen oder zwei Substituenten aufweisen kann, die unabhängig voneinander ausgewählt sind unter C₁-C₆-Alkyl, das gegebenenfalls durch OH, OC₁-C₆-Alkyl, Halogen oder Phenyl substituiert ist, C₁-C₆-Alkoxy, C₂-C₆-Alkenyl, C₂-C₆-Alkinyl, C₃-C₆-Cycloalkyl, Halogen, CN, COR³, NR³R⁴, NO₂, SO₂R³, SO₂NR³R⁴ und Phenyl, das gegebenenfalls durch ein oder zwei Reste substituiert ist, die unabhängig voneinander ausgewählt sind unter C₁-C₆-Alkyl, C₁-C₆-Alkoxy, NR³R⁴, CN, CF₃, CHF₂ oder Halogen;
- R³ und R⁴: unabhängig voneinander für H, C₁-C₆-Alkyl, das gegebenenfalls durch OH, OC₁-C₆-Alkyl, Halogen oder Phenyl substituiert ist, oder Phenyl stehen;
- A: für C₄-C₁₀-Alkylen oder C₃-C₁₀-Alkylen, das wenigstens eine Gruppe Z umfasst, die ausgewählt ist unter O, S, CONR³, COO, CO, C₃-C₆-Cycloalkyl und einer Doppel- oder Dreifachbindung, steht;
- B: einen Rest der folgenden Formeln (a) oder (b) bedeutet: 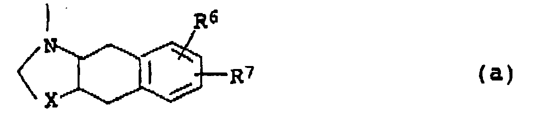 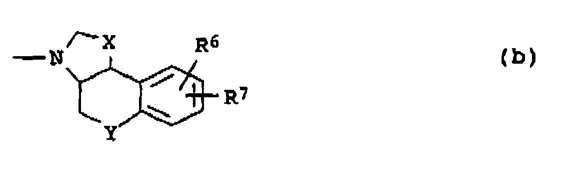
- X: für CH₂ oder CH₂CH₂ steht;
- Y: für CH₂ steht;
- R⁶ und R⁷: unabhängig voneinander ausgewählt sind unter H, C₁-C₆-Alkyl, das gegebenenfalls durch Halogen substituiert ist, OH, C₁-C₆-Alkoxy, C₂-C₆-Alkenyl, Halogen, CN, NO₂, SO₂R³, SO₂NR³R⁴, CONR³R⁴, NHSO₂R³ und NR³R⁴, steht;
sowie deren Salze mit physiologisch verträglichen Säuren.

Bei den erfindungsgemäßen verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als die klassischen Neuroleptika sind, bei denen es sich um D₂-Rezeptorantagonisten handelt. Die Verbindungen sind daher zur Behandlung von Erkrankungen brauchbar, die auf Dopamin-D₃-Liganden ansprechen, d.h., sie sind zur Behandlung von solchen Erkrankungen wirksam, bei denen eine Beeinflussung (Modulation) der Dopamin-D₃-Rezeptoren zur Verbesserung des Krankheitsbildes oder zum Ausheilen der Krankheit führt. Derartige Erkrankungen sind z.B. Erkrankungen des Herz-Kreislaufsystems sowie der Niere, Erkrankungen des zentralen Nervensystems, insbesondere Schizophrenie, affektive Störungen, neurotische Belastungs- und somatoforme Störungen, Psychosen, Parkinson, Aufmerksamkeitsstörungen (attention deficit disorders), Hyperaktivität bei Kindern, Epilepsie, amnestische und kognitive Störungen, wie Lern und Gedächtnisschwäche (impaired cognitive function), Angstzustände, Demenz, Delirium, Persönlichkeitsstörungen, Schlafstörungen (z.B. Restless Legs Syndrome), Störungen des Sexuallebens (Impotenz des Mannes), Eßstörungen und Suchterkrankungen. Außerdem kommen sie zur Behandlung des Schlaganfalls in Betracht.

Zu Suchterkrankungen gehören die durch den Mißbrauch von psychotropen Substanzen, wie Arzneimittel oder Drogen, verursachten psychischen Störungen und Verhaltenssörungen sowie andere Suchterkrankungen, wie beispielsweise die Spielsucht (impulse control disorders not elsewhere classified). Suchterzeugende Substanzen sind beispielsweise: Opioide (z.B. Morphin, Heroin. Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartige Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

vorzugsweise werden die erfindungsgemäßen Verbindungen zur Behandlung von affektiven Störungen; neurotischen, Belastungs- und somatoformen Störungen und Psychosen, z.B. Schizophrenie, eingesetzt.

Im Rahmen der vorliegenden Erfindung besitzen die nachfolgenden Ausdrücke die anschließend angegebenen Bedeutungen:

Alkyl (auch in Resten wie Alkoxy, Alkylthio, Alkylamino etc.) bedeutet eine geradkettige oder verzweigte Alkylgruppe mit 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH, OC₁-C₆-Alkyl, Halogen oder Phenyl. Im Falle eines Halogensubstituenten kann die Alkylgruppe insbesondere 1, 2, 3 oder 4 Halogenatome umfassen, die sich an einem oder mehreren C-Atomen befinden können, vorzugsweise in α- oder ω-Position. Besonders bevorzugt sind CF₃, CHF₂, CF₂Cl oder CH₂F.

Beispiele für eine Alkylgruppe sind Methyl, Ethyl, n-Propyl, isoPropyl, n-Butyl, iso-Butyl, t-Butyl, etc.

Cycloalkyl steht insbesondere für C₃-C₆-Cycloalkyl, wie Cyclopropyl, Cyclobutyl, Cyclopentyl und Cyclohexyl.

Alkylen steht für geradkettige oder verzweigte Reste. Wenn A keine Gruppe z aufweist, umfasst A 4 bis 10 Kohlenstoffatome, bevorzugt 4 bis 8 Kohlenstoffatome. Die Kette zwischen Triazolkern und Gruppe B weist dann mindestens vier Kohlenstoffatome auf. Wenn A wenigstens eine der genannten Gruppen Z aufweist, umfasst A 3 bis 10 Kohlenstoffatome, vorzugsweise 3 bis 8 Kohlenstoffatome.

Wenn die Alkylengruppen wenigstens eine der Gruppen Z umfassen, können diese in der Alkylenkette an beliebiger Stelle oder in Position 1 oder 2 der Gruppe A (vom Triazolrest her gesehen) angeordnet sein. Die Reste CONR² und COO sind vorzugsweise so angeordnet, dass jeweils die Carbonylgruppe dem Triazolring zugewandt ist. Besonders bevorzugt sind Verbindungen der Formel I, worin A für -Z-C₃-C₆-Alkylen, insbesondere -Z-CH₂CH₂CH₂-, -Z-CH₂CH₂CH₂CH₂-, -Z-CH₂CH=CHCH₂-, -Z-CH₂C(CH₃)=CHCH₂-, 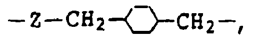 -Z-CH₂CH(CH₃)CH₂- oder für einen linearen -Z-C₇-C₁₀-Alkylenrest steht, wobei Z an den Triazolring gebunden ist. Z steht vorzugsweise für CH₂, O und insbesondere S. Weiterhin bevorzugt steht A für -(CH₂)₄-, -(CH₂)₅-, -CH₂CH₂CH=CHCH₂-, 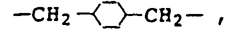 -CH₂CH₂C(CH₃)=CHCH₂- oder -CH₂CH₂CH(CH₃)CH₂-.

Halogen bedeutet F, Cl, Br oder I, vorzugsweise F oder Cl.

R¹ steht vorzugsweise für H, C₁-C₆-Alkyl oder C₃-C₆-Cycloalkyl.

Wenn R² für einen aromatischen Rest steht, handelt es sich vorzugsweise um einen der folgenden Reste: 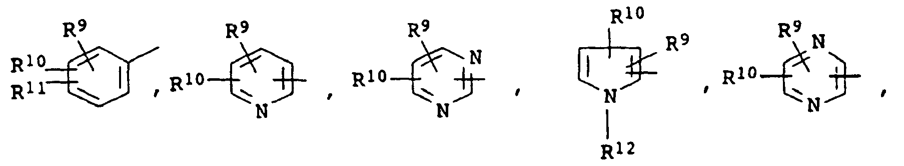 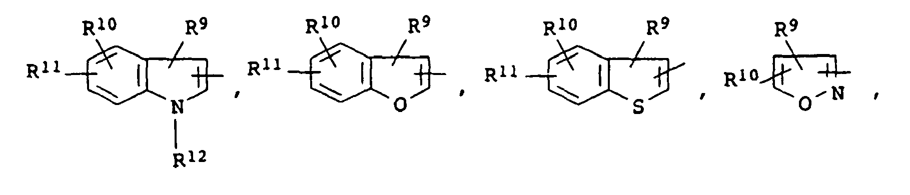 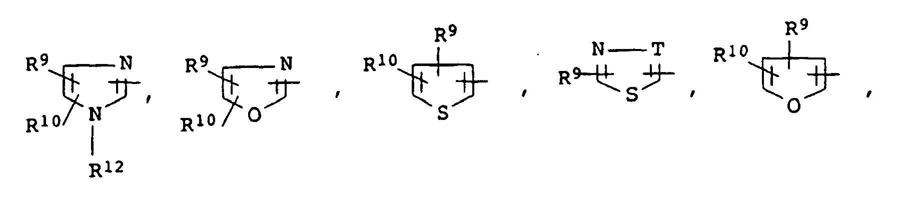 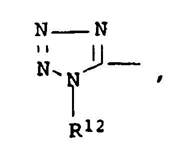 worin
- R⁹ bis R¹¹: für H oder die oben genannten Substituenten des aromatischen Restes Ar¹ stehen,
- R¹²: für H, C₁-C₆-Alkyl oder Phenyl steht und
- T: für N oder CH steht.

Wenn der Phenylrest substituiert ist, stehen die Substituenten vorzugsweise in m- oder p-Stellung.

Besonders bevorzugt handelt es sich bei dem aromatischen Rest um eine Gruppe der Formel: 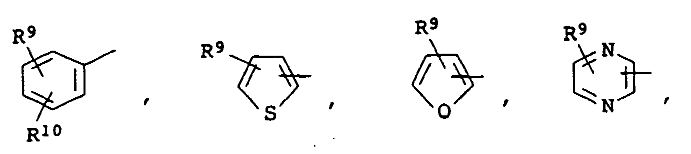 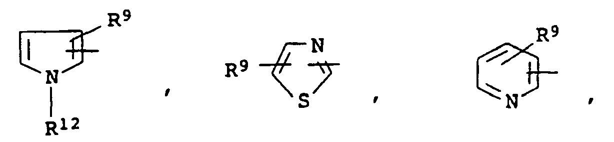 worin R⁹, R¹⁰ und R¹² die oben angegebenen Bedeutungen besitzen. Die angegebenen Phenyl-, Pyridyl-, Thiazolyl- und Pyrrolreste sind insbesondere bevorzugt.

Die Reste R⁹ bis R¹¹ stehen vorzugsweise für H, C₁-C₆-Alkyl, OR³, CN, Phenyl, das gegebenenfalls durch C₁-C₆-Alkyl, C₁-C₆-Alkoxy oder Halogen substituiert ist, CF₃ und Halogen und insbesondere für H, C₁-C₆-Alkyl, OR³ und Halogen. R³ besitzt dabei die oben angegebenen Bedeutungen.

Besonders bevorzugt steht R² für H, C₁-C₆-Alkyl, NR³R⁴ (R³ und R⁴ stehen unabhängig voneinander für H oder C₁-C₆-Alkyl), Phenyl oder einen 5-gliedrigen aromatischen heterocyclischen Rest, der 1 oder 2 Heteroatome aufweist, die unabhängig ausgewählt sind unter N, S und O. Vorzugsweise handelt es sich bei dem heterocyclischen Rest um einen Pyrrol- oder Pyridinrest.

X und/oder Y stehen vorzugsweise für CH₂.

A steht vorzugsweise für C₄-C₁₀-Alkylen oder C₃-C₁₀-Alkylen, das wenigstens eine Gruppe Z umfasst, die ausgewählt ist unter O, S, COO, CO und einer Doppelbindung.

Die Reste R⁶ und R⁷ sind vorzugsweise und unabhängig voneinander ausgewählt unter H, C₁-C₆-Alkyl, OH, C₁-C₆-Alkoxy, C₁-C₆-Alkylthio-C₁-C₆-alkyl, Halogen, CN, NO₂, SO₂R³, SO₂NR³R⁴ und CONR³R⁴. Besonders bevorzugt weist die anellierte Phenylgruppe einen oder zwei Substituenten auf, d.h. einer oder zwei der Reste R⁶, R⁷ und R⁸ stehen für C₁-C₆-Alkyl, OH, Halogen, CN, SO₂NR³R⁴, NO₂ oder CF₃.

Besonders bevorzugt sind die Verbindungen der Formel I, worin
- R¹: für H, C₁C₆-Alkyl oder Phenyl steht,
- R²: für H, C₁-C₆-Alkyl, Phenyl, Thienyl, Furanyl, Pyridyl, Pyrrolyl, Thiazolyl oder Pyrazinyl steht,
- A: für -SC₃-C₁₀-Alkylen, das gegebenenfalls eine Doppelbindung umfassen kann, steht, und
- R⁶ und R⁷: ausgewählt sind unter H, C₁-C₆-Alkyl, C₁-C₆-Alkoxy, Halogen, SO₂NR³R⁴, CN, NO₂ und CF₃.

Die Erfindung umfasst auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Das Verfahren zur Herstellung der Verbindungen der Formel I besteht darin, dass man
a) eine Verbindung der allgemeinen Formel (II) 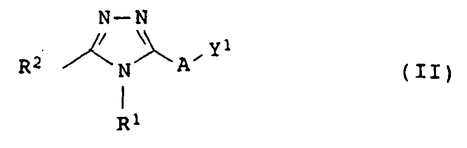 worin Y¹ für eine übliche Abgangsgruppe wie beispielsweise Hal, Alkylsulfonyloxy, Arylsulfonyloxy etc. steht, mit einer Verbindung der allgemeinen Formel (III)

   HB (III)

   umsetzt; oder
b) eine Verbindung der allgemeinen Formel (IV) 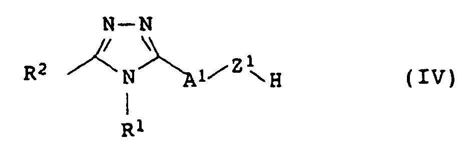 worin Z¹ für O oder S und A¹ für C₁-C₁₀-Alkylen oder eine Bindung steht, mit einer Verbindung der allgemeinen Formel (V)

   Y¹ - A² - B (V)

   wobei Y¹ die oben angegebene Bedeutung besitzt und A² für C₂-C₁₀-Alkylen steht, wobei A¹ und A² zusammen 3 bis 10 C-Atome aufweisen und A¹ und/oder A² gegebenenfalls wenigstens eine Gruppe Z umfassen, umsetzt; oder
c) eine Verbindung der allgemeinen Formel (VI) 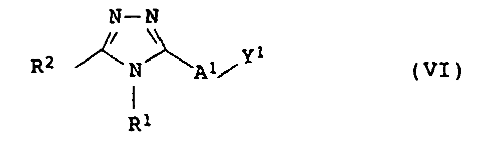 worin Y¹ und A¹ die oben angegebenen Bedeutungen besitzen, mit einer Verbindung der allgemeinen Formel (VII)

   H - Z¹ - A - B (VII)

   worin Z¹ die oben angegebenen Bedeutungen besitzt, umsetzt; oder
d) eine Verbindung der allgemeinen Formel (VIII) 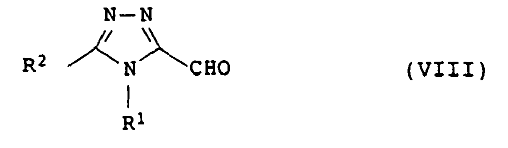 mit literaturbekannten Reagenzien, wie z. B. 1,3-Propandithiol, KCN/wasser, TMSCN (Trimethylsilylcyanid) oder KCN/Morpholin, wie z. B. beschrieben in
   Albright Tetrahedron, 1983, 39, 3207 oder
   D. Seebach Synthesis 1969, 17 und 1979, 19 oder
   H. Stetter Angew. Chem. Int. Ed. 1976, 15, 639 oder
   van Niel et al. Tetrahedron 1989, 45, 7643
   Martin et al. Synthesis 1979, 633,
   zu den Produkten (VIIIa) (exemplarisch mit 1,3-Propandithiol) 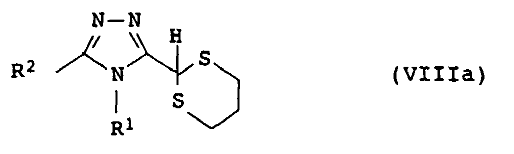 umpolt und anschließend mit Verbindungen der allgemeinen Formel (IX)

   Y¹ - A³ - B (IX)

   wobei Y¹ die oben angegebene Bedeutung besitzt und A³ für C₃-C₉-Alkylen steht, das eine Gruppe Z enthalten kann, kettenverlängert, wobei man nach Entschützen oder Reduktion
   Verbindungen der Formel (Ia) 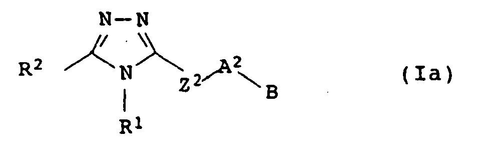 worin Z² für CO oder eine Methylengruppe steht und Z² und A² zusammen 4 bis 10 C-Atome aufweisen, erhält, oder
e) eine Verbindung der allgemeinen Formel (VIII) mit einer Verbindung der allgemeinen Formel (X)

   Y² - A - B (X)

   worin Y² für ein Phosphoran oder einen Phosphonsäureester steht, analog zu üblichen Methoden, wie zum Beispiel beschrieben in Houben Weyl "Handbuch der Organischen Chemie" 4. Auflage, Thieme Verlag Stuttgart, Band V/1b S.383 ff oder Bd V/1c S.575 ff, umsetzt, oder
f) eine Verbindung der allgemeinen Formel (XI) 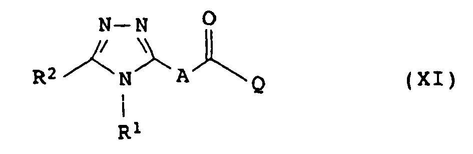
worin Q für H oder OH steht, mit einer Verbindung der Formel III unter reduktiven Bedingungen analog literaturbekannten Methoden, wie z. B. beschrieben in J. Org. Chem. 1986, 50, 1927; oder WO 92/20655 umsetzt.

Das Verfahren zur Herstellung einer Verbindung der Formel I, worin A die Gruppe COO oder CONR³ umfasst, besteht darin, dass man eine Verbindung der allgemeinen Formel (XII) 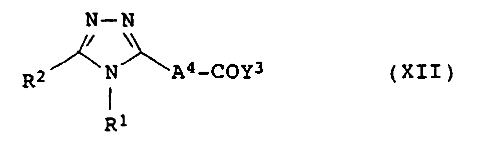 worin Y³ für OH, OC₁-C₄-Alkyl, Cl oder zusammen mit CO für eine aktivierte Carboxylgruppe, und A⁴ für C₀-C₉-Alkylen steht, mit einer Verbindung der Formel (XIII)

B - A - Z³ (XIII)

worin Z³ für OH oder NHR³ steht,
umsetzt.
Verbindungen des Typs (XIV) 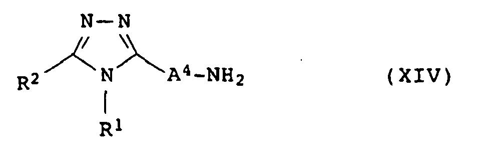 können synthetisiert werden durch Alkylierung von Verbindungen der Formel (IV) mit Verbindungen der allgemeinen Formel (XV), 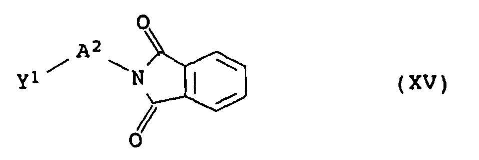 zu Verbindungen der Formel (XVI), 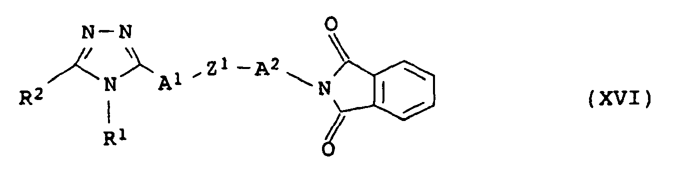 sich anschließender Hydrazinolyse zu Verbindungen des Typs (XVII)

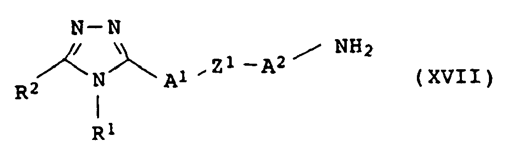

Verbindungen der allgemeinen Formel XVII können auch erhalten werden durch Umsetzung von Verbindungen der Formel II mit Aziden, wie z.B. Natriumazid, und anschließender Reduktion, wie z. B. beschrieben in
H. Staudinger, Helv. Chim. Acta 1919, 2, 635 oder
R. Carrie, Bull. Chem. Soc. Fr. 1985, 815.

Verbindungen der allgemeinen Formeln B-H können hergestellt werden wie z.B. beschrieben in
S. Smith et al., Bioorg. Med. Chem. Lett. 1998, 8, 2859;
WO 97/47602, WO 920655 und WO98/24791 bzw.
J. Med. Chem. 1987, 30, 2111 und 2208.

Die Verbindungen des Typs der Formel (IV) sind entweder bekannt oder können nach bekannten Verfahren hergestellt werden, wie z.B. beschrieben in A.R. Katritzky, C.W. Rees (ed.) "Comprehensive Heterocyclic Chemistry", Pergamon Press, oder "The Chemistry of Heterocyclic Compounds" J. Wiley & Sons Inc. NY und der dort zitierten Literatur oder in S. Kubota et al. Chem. Pharm. Bull 1975, 23, 955 oder Vosilevskii et al. Izv. Akad. Nauk. SSSR Ser. Khim 1975, 23, 955.

In den obigen Formeln besitzen R¹, R², R⁶, R⁷, A, B, X und Y die im Zusammenhang mit Formel I angegebenen Bedeutungen.

Die Herstellung der erfindungsgemäßen Verbindungen und der Ausgangsmaterialien und der Zwischenprodukte kann auch analog zu den in den eingangs genannten Patentpublikationen beschriebenen Methoden erfolgen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ester, wie Ethylacetat, Ether, wie Diethylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Ethanol oder Butanol.

Gewünschtenfalls arbeitet man in Gegenwart eines säurebindenden Mittels. Geeignete säurebindende Mittel sind anorganische Basen, wie Natrium- oder Kaliumcarbonat, Natrium- oder Kaliumhydrogencarbonat, Natriummethylat, Natriumethylat, Natriumhydrid oder metallorganische Verbindungen, wie Butyllithium- oder Alkylmagnesium-Verbindungen, oder organische Basen, wie Triethylamin oder Pyridin. Letztere können gleichzeitig als Lösungsmittel dienen.

Das Verfahren (f) erfolgt unter reduzierenden Bedingungen, z.B. unter Verwendung von Natriumborhydrid, Natriumcyanoborhydrid oder Triacetoxyborhydrid, gegebenenfalls in saurem Medium oder in Gegenwart einer Lewissäure, wie z.B. Zinkchlorid oder mittels katalytischer Hydrierung.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder Überführen in eine Säureadditionsverbindung.

Die Säureadditionssalze werden in üblicher weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den wirkstoff normalerweise in einer Menge von 1 bis 99 Gew.-%.

Die nachfolgenden Beispiele dienen zur Erläuterung der Erfindung ohne sie zu begrenzen.

### Beispiel 1

### (cis/trans) 9-Brom-3-{3-[(4-methyl-5-phenyl-4H-1,2,4-triazol-3-yl)sulfanyl]propyl}-2,3,3a,4,5,9b-hexahydro-1H-benzo[e]indol

### Synthese der Ausgangsstoffe

### 1A (8-Bromo-2-oxo-1,2,3,4-tetrahydronaphthalen-1-yl)-essigsäuremethylester

Eine Lösung von 13.5 g (60 mmol) 8-Brom-tetralon in 470 ml THF wurde bei -30°C unter Schutzgasatmosphäre mit 33 ml 2M Lithiumdiisopropylamin versetzt, nachgerührt und dann eine Lösung von 11 g (72 mmol) Methylbromacetat in 100 ml THF zugetropft. Nach 18 h bei Raumtemperatur wurde zur Aufarbeitung bei 0°C mit 10 ml konzentrierter Salzsäure versetzt. Die Lösemittel entfernte man im Vakuum, nahm den Rückstand in Wasser auf und extrahierte mit Essigsäureethylester. Die vereinigten organischen Phasen wurden über Natriumsulfat getrocknet, filtriert und eingeengt; der Rückstand wurde säulenchromatographisch (Kieselgel, Laufmittel: Methylenchlorid) gereinigt.

Ausbeute: 13.7g (46 mmol); 77% der Theorie
C₁₃H₁₃BrO₃ (297.2) MS: 296/298 [M⁺]

### 1B (cis/trans) 9-Brom-3-{3-[(4-methyl-5-phenyl-4H-1,2,4-triazol-3-yl)sulfanyl]propyl}-1,3,3a,4,5,9b-hexahydro-2H-benzo[e]indol-2-on

Eine Lösung von 0.9 g (3.6 mmol) 3-[(4-Methyl-5-phenyl-4H-1,2,4-triazol-3-yl)sulfanyl]propylamin und 0.7 g (2.4 mmol) der vorstehenden Verbindung in 10 ml THF/Methanol = 1/1 wurde bei 0°C mit 0.8 ml Eisessig (pH 4-5) dann mit 0.2 mg (2.4 mmol) Natriumcyanoborhydrid versetzt und 72 Stunden bei Raumtemperatur gerührt. Zur Aufarbeitung versetzte man mit 20%-iger Natronlauge, engte im Vakkum ein und nahm den Rückstand mit Dichlormethan auf. Die organische Phase wurde mit Wasser gewaschen, über Natriumsulfat getrocknet, filtriert und eingeengt worauf eine säulenchromatographische Reinigung des Rückstandes (Kieselgel, Laufmittel: Methylenchlorid mit 3-5% Methanol) erfolgte.

Ausbeute: 0.6 g (1.1 mmol); 47% der Theorie
¹H-NMR (CDCl3): δ = 1.8-2.3 (m, 6H); 2,7 (mbr, 2H); 3.1-3.4 (m, 4H); 3.6 (s, 3H); 3.8-4.0 (m, 2H); 4.1 (m, 1H); 7.1 (m, 2H); 7.4 (d, 1H); 7.5 (m, 3H); 7.7 (m, 2H).
C₂₄H₂₅BrN₄OS (497.5)

### Darstellung des Endproduktes

1.9 ml einer 1M Boran-THF-Lösung wurden unter Eiskühlung und Schutzgas mit 560 mg (1.1 mmol) der unter 1B hergestellten und in 2ml THF gelösten Verbindung versetzt und 1 Stunde zum Sieden erhitzt. Nach beendeter Reaktion gab man 1 ml 10%-ige Salzsäure zu, engte ein, nahm den Rückstand in Wasser auf, stellte alkalisch und extrahierte mit Dichlormethan. Die vereinigte organischen Phasen wurden über Natriumsulfat getrocknet, filtriert und eingeengt. Das Rohprodukt reinigte man chromatographisch auf (Kieselgel, Laufmittel: Methylenchlorid mit 3-5% Methanol).

Ausbeute: 40 mg (0.1 mmol); 8% der Theorie
¹H-NMR (CDCl3): δ = 1.3 - 1.5 (m, 2H); 1.9 (m, 1H); 2.0 (m, 2H); 2.2 (m, 1H); 2.4 - 2.5 (m, 2H); 2.6 (m, 1H); 2.8 - 3.0 (m, 3H); 3.1 (t, 1H); 3.2 - 3.4 (2m, 2H); 3.6 (m, 4H); 6.9 (t, 1H); 7.0 (d, 1H); 7.4 (d, 1H); 7.5 (m, 3H); 7.7 (m, 2H).
C₂₄H₂₇BrN₄S (482.9)

In prinzipiell analoger Weise wurde dargestellt:

### Beispiel 2

### (cis/trans) 7-tert.-Butyl-3-{3-[(4-methyl-5-phenyl-4H-1,2,4-triazol-3-yl)sulfanyl]propyl}-2,3,3a,4,5,9b-hexahydro-1H-henzo[e]indol

C₂₈H₃₆N₄S (460.7) MS: 461 [M⁺]

### Beispiel 3

### (cis/trans) 9-Brom-3-{3-[(4-methyl-5-(thien-3-yl)-4H-1,2,4-triazol-3-yl)sulfanyl]propyl}-2,3,3a,4,5,9b-hexahydro-1H-benzo[e]indol

### Beispiel 4

### (cis/trans) 3-{3-[(4-methyl-5-phenyl-4H-1,2,4-triazol-3-yl)butyl}-2,3,3a,4,5,9b-hexahydro-1H-benzo[e]indol

In prinzipiell analoger weise können hergestellt werden:

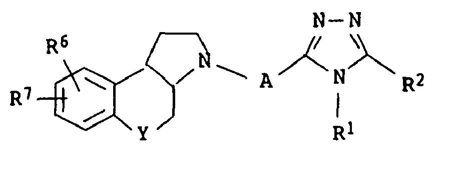

**Tabelle 1:**

| Bsp | R¹ | R² | A | Y | R⁶ | R⁷* |
|---|---|---|---|---|---|---|
| 5 | Et | 2,5-Di-methyl-furanyl-3- | S-(CH₂)₃- | CH₂ | 7-tert-butyl | H |
| 6 | Me | 2-Pyrazinyl- | O-(CH₂)₃- | CH₂ | H | |
| 7 | Propyl | 3-Br-Pyridin-5-yl- | S-(CH₂)₃- | CH₂ | 7-carboxamido | |
| 8 | Phenyl | Cyano | S-(CH₂)₃- | CH₂ | H | |
| 9 | Ethyl | 3-Jod-phenyl | (CH₂)₄- | O | 7-tert-butyl | |
| 10 | Me | 3-Pyrrolyl | S-(CH₂)₃- | CH₂ | 8-fluor | |
| 11 | butyl | 2,5-Di-methyl-furanyl-3- | S-(CH₂)₃- | CH₂ | 7-methoxy | 8-methoxy |
| 12 | Me | 2-Pyrazinyl- | S-(CH₂)₃- | CH₂ | 7-tert-butyl | |
| 13 | cycPropyl | Pyridin-4-yl- | S-(CH₂)₃- | CH₂ | 8-fluor | |
| 14 | Ethyl | 4-Methylthiazol-5-yl | S-CH₂-C(CH₃)=CH-CH₂- | CH₂ | 7-tert-butyl | |
| 15 | Me | 4-Methoxyphenyl | (CH₂)₄- | CH₂ | 9-fluor | |
| 16 | Me | 4-Jod-phenyl | S-(CH₂)₃- | CH₂ | 7-tert-butyl | |
| 17 | Me | 2-Thienyl | (CH₂)₄- | CH₂ | H | |
| 18 | Propyl | 4-Imidazolyl- | S-(CH₂)₃- | CH₂ | 7-carboxamido | |
| 19 | Me | 2,5-Di-methyl-furanyl-3- | S-(CH₂)₃- | CH₂ | H | |
| 20 | Propyl | 4-Methylthiazol-5-yl | CONH-(CH₂)₄- | CH₂ | 7-methyl | |
| 21 | Me | Phenyl | S-CH₂-C(=CH₂)-CH₂ | O | 8-brom | |
| 22 | Me | N-Methyl-2-Pyrrolyl- | (CH₂)₄- | O | 9-methyl | |
| 23 | Me | Tetrazolyl- | S-(CH₂)₃- | CH₂ | 8-fluor | |
| 24 | Me | 2-Me-4-Oxazolyl- | S-(CH₂)₃- | O | 7-tert-butyl | |
| 25 | Me | Oxadiazol-2-yl | S-(CH₂)₃- | CH₂ | H | |
| 26 | Me | 3-Cyano-phenyl | S-(CH₂)₃- | CH₂ | 6-methoxy | |
| 27 | isoPropyl | 3-Br-Pyridin-5-yl- | S-(CH₂)₃- | CH₂ | 8-trifluormethoxy | |
| 28 | Me | 2-Thienyl | S-CH₂-C(=CH₂)-CH₂ | CH₂ | 8-fluor | |
| 29 | Me | 2-Aminothiazol-4yl- | S-(CH₂)₃- | CH₂ | 6-methoxy | |
| 30 | Me | 3-Cyano-phenyl | S-(CH₂)₃- | O | H | |
| 31 | Me | 3-Thienyl | S-(CH₂)₃- | CH₂ | 7-tert-butyl | |
| 32 | Me | 4-Methylthiazol-5-yl | S-(CH₂)₃- | O | 7-nitro | |
| 33 | Me | Cyclohexyl- | S-(CH₂)₃- | CH₂ | 8-fluor | |
| 34 | isoPropyl | 2-Aminothiazol-4yl- | S-(CH₂)₃- | CH₂ | 8-trifluormethoxy | |
| 35 | Me | 3-Jod-phenyl | (CH₂)₄- | O | 7-tert-butyl | |
| 36 | butyl | Pyridin-4-yl- | S-(CH₂)₃- | CH₂ | 7-methoxy | 8-methoxy |
| 37 | Me | 4-Methoxyphenyl | S-(CH₂)₃- | CH₂ | 7-cyano | |
| 38 | Hexyl | 4-Imidazolyl- | S-(CH₂)₃- | CH₂ | 6-methoxy | |
| 39 | Ethyl | Pyridin-3-yl- | S-(CH₂)₆- | CH₂ | 7-tert-butyl | |
| 40 | Me | 4-Methoxyphenyl | (CH₂)₄- | O | 9-fluor | |
| 41 | Propyl | N-Methyl-2-Pyrrolyl- | CONH-(CH₂)₄- | CH₂ | 8-trifluormethoxy | |
| 42 | cycPropyl | 4-Methoxyphenyl | S-(CH₂)₃- | CH₂ | 8-fluor | |
| 43 | Me | N-Methyl-2-Pyrrolyl- | S-(CH₂)₃- | CH₂ | 7-nitro | |
| 44 | Me | 3-Jod-phenyl | O-(CH₂)₃- | O | H | |
| 45 | Me | N-Propyl-tetrazolyl- | S-(CH₂)₃- | O | H | |
| 46 | isoButyl | Oxadiazol-2-yl | S-(CH₂)₃- | O | 7-tert-butyl | |
| 47 | Ethyl | Pyridin-3-yl- | (CH₂)₄- | O | H | |
| 48 | Me | 2-Thienyl | S-(CH₂)₃- | O | 7-nitro | |
| 49 | Me | Phenyl | CO-CH₂-C(=CH₂)-CH₂ | O | 7-tert-butyl | |
| 50 | Me | Pyridin-3-yl- | S-(CH₂)₃- | CH₂ | 6-methoxy | |
| 51 | Me | N-Methyl-2-Pyrrolyl- | S-(CH₂)₃- | CH₂ | H | |
| 52 | Me | Pyridin-3-yl- | S-(CH₂)₃- | O | 7-cyano | |
| 53 | butyl | 3-Cyano-phenyl | S-(CH₂)₃- | CH₂ | 7-methoxy | 8-methoxy |
| 54 | Me | 2-Thienyl | S-(CH₂)₈- | O | 6-methoxy | |
| 55 | Me | 2-Pyrazinyl- | S-(CH₂)₃- | O | 7-nitro | |
| 56 | Ethyl | 2-Thienyl | (CH₂)₄- | O | H | |
| 57 | Ethyl | Phenyl | S-CH₂-C(CH₃)=CH-CH₂- | O | 7-tert-butyl | |
| 58 | Me | 4-Methoxyphenyl | S-(CH₂)₃- | CH₂ | 7-tert-butyl | |
| 59 | Me | 3-Br-Pyridin-5-yl- | S-(CH₂)₃- | CH₂ | H | |
| 60 | Me | N-Methyl-2-Pyrrolyl- | S-(CH₂)₃- | O | H | |
| 61 | Me | N-Propyl-tetrazolyl- | S-(CH₂)₃- | CH₂ | 8-trifluormethoxy | |
| 62 | butyl | 3-Br-Pyridin-5-yl- | S-(CH₂)₃- | CH₂ | 7-methoxy | 8-methoxy |
| 63 | Ethyl | Phenyl | S-CH₂-C(CH₃)=CH-CH₂- | O | 7-sulfonamido | |
| 64 | Me | 2-Thienyl | S-(CH₂)₃- | CH₂ | 7-sulfonamido | |
| 65 | Me | Pyridin-3-yl- | S-(CH₂)₈- | CH₂ | 8-trifluormethoxy | |
| 66 | Me | 3-Jod-phenyl | S-(CH₂)₃- | CH₂ | 8-fluor | |
| 67 | Me | Phenyl | O-(CH₂)₄- | O | 8-iod | |
| 68 | Me | Pyridin-3-yl- | S-(CH₂)₈- | O | 8-trifluormethoxy | |
| 69 | isoPropyl | 3-Cyano-phenyl | S-(CH₂)₃- | CH₂ | 8-trifluormethoxy | |
| 70 | butyl | N-Propyl-tetrazolyl- | S-(CH₂)₃- | CH₂ | 7-methoxy | 8-methoxy |
| 71 | Ethyl | Pyridin-3-yl- | S-CH₂-CH=CH-CH₂- | CH₂ | 8-fluor | |
| 72 | Me | 3-Br-Pyridin-5-yl- | S-(CH₂)₃- | O | H | |
| 73 | Phenyl | Methylamino | S-(CH₂)₃- | CH₂ | 8-fluor | |
| 74 | Ethyl | Phenyl | CO-(CH₂)₃- | O | H | |
| 75 | Me | Phenyl | S-(CH₂)₁₀- | CH₂ | 7-methyl | |
| 76 | cycPropyl | 4-Imidazolyl- | S-(CH₂)₃- | CH₂ | 8-fluor | |
| 77 | Me | 2-Thienyl | S-(CH₂)₃- | O | H | |
| 78 | Me | 3-Jod-phenyl | O-(CH₂)₃- | CH₂ | H | |
| 79 | Et | 3-Benzthienyl- | S-(CH₂)₃- | CH₂ | 7-tert-butyl | |
| 80 | Me | 4-Methoxyphenyl | S-(CH₂)₃- | CH₂ | 7-nitro | |
| 81 | Ethyl | Phenyl | S-(CH₂)₃- | O | 7-nitro | |
| 82 | Ethyl | 2-Thienyl | CO-(CH₂)₃- | CH₂ | H | |
| 83 | cycPropyl | 3-Cyano-phenyl | S-(CH₂)₃- | CH₂ | 8-fluor | |
| 84 | Propyl | Phenyl | CONH-(CH₂)₅- | CH₂ | 7-methyl | |
| 85 | Me | 2,5-Di-methyl-furanyl-3- | S-(CH₂)₃- | O | H | |
| 86 | Et | N-Propyl-tetrazolyl- | S-(CH₂)₃- | CH₂ | 7-tert-butyl | |
| 87 | Ethyl | 2-Aminothiazol-4yl- | S-(CH₂)₃- | | H | |
| 88 | Me | Tetrazolyl- | S-(CH₂)₃- | CH₂ | H | |
| 89 | Propyl | Phenyl | COO-(CH₂)₄- | CH₂ | 7-methyl | |
| 90 | Propyl | 2-Pyrazinyl- | COO-(CH₂)₄- | CH₂ | 8-trifluormethoxy | |
| 91 | Propyl | Pyridin-3-yl- | CONH-(CH₂)₄- | CH₂ | 7-methyl | |
| 92 | Me | Oxadiazol-2-yl | S-(CH₂)₃- | CH₂ | 8-fluor | |
| 93 | butyl | 3-Benzthienyl- | S-(CH₂)₃- | CH₂ | 7-methoxy | 8-methoxy |
| 94 | Me | 4-Methylthiazol-5-yl | (CH₂)₄- | CH₂ | 9-mehyl | |
| 95 | Me | 4-Methylthiazol-5-yl | (CH₂)₄- | O | 9-methyl | |
| 96 | Me | 3-Jod-phenyl | S-(CH₂)₃- | CH₂ | H | |
| 97 | Me | 2-Thienyl | (CH₂)₄- | O | H | |
| 98 | isoPropyl | 5-Methyl imidazol-4-yl- | S-(CH₂)₃- | O | 8-trifluormethoxy | |
| 99 | Me | N-Methyl-2-Pyrrolyl- | S-(CH₂)₃- | O | 7-cyano | |
| 100 | Me | Phenyl | S-(CH₂)₃- | CH₂ | 7-cyano | |
| 101 | isoButyl | Tetrazolyl- | S-(CH₂)₃- | O | H | |
| 102 | Me | Phenyl | CO-CH₂-C(=CH₂)-CH₂ | CH₂ | 7-tert-butyl | |
| 103 | Me | 2-Pyrazinyl- | S-(CH₂)₃- | CH₂ | 6-methoxy | |
| 104 | Me | 2-Pyrazinyl- | S-(CH₂)₃- | CH₂ | 7-nitro | |
| 105 | Ethyl | Pyridin-3-yl- | S-CH₂-CH=CH-CH₂- | O | 8-fluor | |
| 106 | Me | 4-Methylthiazol-5-yl | S-(CH₂)₃- | O | H | |
| 107 | Me | 2-Pyrazinyl- | (CH₂)₄- | O | 7-methyl | |
| 108 | Phenyl | 4-Imidazolyl- | S-(CH₂)₃- | O | H | |
| 109 | Me | Pyridin-4-yl- | S-(CH₂)₃- | CH₂ | 8-trifluormethoxy | |
| 110 | Me | 2-Thienyl | S-(CH₂)₇- | CH₂ | 8-trifluormethoxy | |
| 111 | Hexyl | 3-Benzthienyl- | S-(CH₂)₃- | CH₂ | 6-methoxy | |
| 112 | cycPropyl | Amino | S-(CH₂)₃- | CH₂ | 8-fluor | |
| 113 | Me | 2-Thienyl | S-(CH₂)₃- | O | 7-cyano | |
| 114 | Me | Oxadiazol-2-yl | S-(CH₂)₃- | CH₂ | 7-tert-butyl | |
| 115 | Ethyl | 4-Methylthiazol-5-yl | S-CH₂-C(=CH₂)-CH₂ | O | 7-sulfonamido | |
| 116 | Me | 2-Thienyl | S-(CH₂)₃- | CH₂ | 8-fluor | |
| 117 | Me | Methylamino | S-(CH₂)₃- | CH₂ | H | |
| 118 | Me | 3-Pyrrolyl | S-(CH₂)₃- | CH₂ | 7-tert-butyl | |
| 119 | Me | N-Methyl-2-Pyrrolyl- | S-(CH₂)₃- | CH₂ | 7-sulfonamido | |
| 120 | butyl | 2-Aminothiazol-4yl- | S-(CH₂)₃- | CH₂ | 7-methoxy | 8-methoxy |
| 121 | Me | N-Methyl-2-Pyrrolyl- | S-(CH₂)₃- | O | 7-nitro | |
| 122 | isoPropyl | Phenyl | S-(CH₂)₃- | O | 7-sulfonamido | |
| 123 | Me | 2-Me-4-Oxazolyl- | S-(CH₂)₃- | CH₂ | 6-methoxy | |
| 124 | cycPropyl | Methylamino | S-(CH₂)₃- | CH₂ | 7-tert-butyl | |
| 125 | isoPropyl | Cyano | S-(CH₂)₃- | CH₂ | 6-methoxy | |
| 126 | Me | Phenyl | S-(CH₂)₃- | CH₂ | 6-methoxy | |
| 127 | Propyl | 3-Cyano-phenyl | S-(CH₂)₃- | CH₂ | 7-carboxamido | |
| 128 | Propyl | Phenyl | CONH-(CH₂)₅- | O | 7-methyl | |
| 129 | Ethyl | 3-Jod-phenyl | S-(CH₂)₃- | CH₂ | 7-methoxy | 8-methoxy |
| 130 | Ethyl | 4-Methylthiazol-5-yl | S-(CH₂)₆- | 0 | 7-cyano | |
| 131 | Me | N-Methyl-2-Pyrrolyl- | S-(CH₂)₃- | CH₂ | 7-methoxy | 8-methoxy |
| 132 | Me | 2-Aminothiazol-4yl- | S-(CH₂)₃- | CH₂ | H | |
| 133 | Me | Oxadiazol-2-yl | S-(CH₂)₃- | CH₂ | 7-methoxy | 8-methoxy |
| 134 | Me | 2-Me-4-Oxazolyl- | S-(CH₂)₃- | CH₂ | 7-methoxy | 8-methoxy |
| 135 | Me | Pyridin-3-yl- | (CH₂)₄- | CH₂ | H | |
| 136 | Ethyl | Phenyl | S-CH₂-C(CH₃)=CH-CH₂- | CH₂ | 7-tert-butyl | |
| 137 | Me | Phenyl | (CH₂)₄- | O | H | |
| 138 | Et | 4-Imidazolyl- | S-(CH₂)₃- | CH₂ | 7-tert-butyl | |
| 139 | isoPropyl | 3-Benzthienyl- | S-(CH₂)₃- | CH₂ | 8-trifluormethoxy | |
| 140 | Me | 2-Pyrazinyl- | S-(CH₂)₃- | O | 7-cyano | |
| 141 | Hexyl | Amino | S-(CH₂)₃- | CH₂ | 6-methoxy | |
| 142 | Me | 5-Methyl imidazol-4-yl- | S-(CH₂)₃- | CH₂ | 6-methoxy | |
| 143 | Me | Pyridin-3-yl- | S-(CH₂)₃- | CH₂ | 7-tert-butyl | |
| 144 | Me | Pyridin-3-yl- | S-(CH₂)₃- | CH₂ | 8-fluor | |
| 145 | cycPropyl | N-Propyl-tetrazolyl- | S-(CH₂)₃- | CH₂ | 8-fluor | |
| 146 | Me | 2-Me-4-Oxazolyl- | S-(CH₂)₃- | CH₂ | H | |
| 147 | Me | 3-Jod-phenyl | S-(CH₂)₃- | CH₂ | 7-nitro | |
| 148 | Propyl | 4-Methoxyphenyl | COO-(CH₂)₃- | CH₂ | 7-sulfonamido | |
| 149 | Me | Pyridin-3-yl- | (CH₂)₄- | O | H | |
| 150 | Me | 2-Thienyl | O-(CH₂)₄- | CH₂ | 7-tert-butyl | |
| 151 | butyl | 5-Methyl imidazol-4-yl- | S-(CH₂)₃- | CH₂ | 7-methoxy | 8-methoxy |
| 152 | isoButyl | Methylamino | S-(CH₂)₃- | O | H | |
| 153 | Hexyl | N-Propyl-tetrazolyl- | S-(CH₂)₃- | CH₂ | 6-methoxy | |
| 154 | Phenyl | Amino | S-(CH₂)₃- | O | H | |
| 155 | Me | Phenyl | CO-CH₂-C(=CH₂)-CH₂ | O | 7-tert-butyl | |
| 156 | Me | 2-Pyrazinyl- | S-(CH₂)₃- | O | H | |
| 157 | Me | 3-Pyrrolyl | S-(CH₂)₃- | CH₂ | 6-methoxy | |
| 158 | Me | 2-Me-4-Oxazolyl- | S-(CH₂)₃- | CH₂ | 8-fluor | |
| 159 | Me | Phenyl | S-(CH₂)₃- | O | H | |
| 160 | Ethyl | Pyridin-3-yl- | S-(CH₂)₆- | O | 7-tert-butyl | |
| 161 | Me | 3-Jod-phenyl | S-(CH₂)₃- | CH₂ | 7-sulfonamido | |
| 162 | Me | 3-Pyrrolyl | S-(CH₂)₃- | O | H | |
| 163 | isoPropyl | 3-Pyrrolyl | S-(CH₂)₃- | CH₂ | 8-trifluormethoxy | |
| 164 | Me | Pyridin-3-yl- | S-(CH₂)₃- | O | 7-tert-butyl | |
| 165 | Me | 2-Thienyl | S-(CH₂)₃- | O | 7-nitro | |
| 166 | butyl | 4-Imidazolyl- | S-(CH₂)₃- | CH₂ | 7-methoxy | 8-methoxy |
| 167 | Me | Tetrazolyl- | S-(CH₂)₃- | CH₂ | 7-tert-butyl | |
| 168 | Me | Phenyl | O-(CH₂)₄- | CH₂ | 7-tert-butyl | |
| 169 | Me | Phenyl | S-(CH₂)₃- | CH₂ | 7-methoxy | 8-methoxy |
| 170 | Me | N-Methyl-2-Pyrrolyl- | (CH₂)₄- | CH₂ | 9-methyl | |
| 171 | Me | 3-Jod-phenyl | (CH₂)₄- | CH₂ | 7-tert-butyl | |
| 172 | Me | 5-Methyl imidazol-4-yl- | S-(CH₂)₃- | CH₂ | 8-fluor | |
| 173 | Me | 2-Aminothiazol-4yl- | S-(CH₂)₃- | CH₂ | 7-tert-butyl | |
| 174 | Me | 4-Methoxyphenyl | S-(CH₂)₃- | 0 | 7-nitro | |
| 175 | Me | Pyridin-4-yl- | S-(CH₂)₃- | O | H | |
| 176 | Me | 2-Thienyl | S-(CH₂)₃- | CH₂ | 7-cyano | |
| 177 | Me | 2-Thienyl | S-(CH₂)₃- | CH₂ | 7-methoxy | 8-methoxy |
| 178 | Me | 2-Pyrazinyl- | O-(CH₂)₃- | O | H | |
| 179 | isoPropyl | 5-Methyl imidazol-4-yl- | S-(CH₂)₃- | CH₂ | 8-trifluormethoxy | |
| 180 | Me | Pyridin-3-yl- | S-(CH₂)₃- | O | 7-nitro | |
| 181 | Me | Phenyl | S-CH₂-C(=CH₂)-CH₂ | CH₂ | 8-brom | |
| 182 | Me | Tetrazolyl- | S-(CH₂)₃- | CH₂ | 7-methoxy | 8-methoxy |
| 183 | Propyl | 2-Aminothiazol-4yl- | S-(CH₂)₃- | CH₂ | 7-carboxamido | |
| 184 | Me | 3-Br-Pyridin-5-yl- | S-(CH₂)₃- | CH₂ | 8-fluor | |
| 185 | Me | N-Methyl-2-Pyrrolyl- | S-CH₂-CH=CH-CH₂- | CH₂ | 8-fluor | |
| 186 | Propyl | 2-Thienyl | COO-(CH₂)₄- | CH₂ | 6-methoxy | |
| 187 | Me | 2-Thienyl | S-(CH₂)₃- | CH₂ | 6-methoxy | |
| 188 | Propyl | 3-cyano-phenyl | CONH-(CH₂)₅- | CH₂ | 7-methyl | |
| 189 | Propyl | Pyridin-4-yl- | S-(CH₂)₃- | CH₂ | 7-carboxamido | |
| 190 | Me | 3-Cyano-phenyl | S-(CH₂)₃- | CH₂ | H | |
| 191 | isoPropyl | 4-Methoxyphenyl | S-(CH₂)₃- | O | 7-sulfonamido | |
| 192 | Me | Phenyl | S-(CH₂)₃- | CH₂ | 8-trifluormethoxy | |
| 193 | Me | 2-Pyrazinyl- | S-(CH₂)₃- | CH₂ | 7-sulfonamido | |
| 194 | Ethyl | Phenyl | S-(CH₂)₃- | CH₂ | 7-nitro | |
| 195 | Phenyl | 3-Benzthienyl- | S-(CH₂)₃- | CH₂ | H | |
| 196 | Me | 2-Pyrazinyl- | S-(CH₂)₃- | CH₂ | 8-fluor | |
| 197 | Me | 3-Jod-phenyl | S-(CH₂)₃- | CH₂ | 7-cyano | |
| 198 | Ethyl | 4-Methylthiazol-5-yl | S-CH₂-C(=CH₂)-CH₂ | CH₂ | 7-sulfonamido | |
| 199 | Me | 3-Cyano-phenyl | S-(CH₂)₃- | CH₂ | 7-tert-butyl | |
| 200 | cycPropyl | 2-Me-4-Oxazolyl- | S-(CH₂)₃- | CH₂ | 7-tert-butyl | |
| 201 | Me | 3-Jod-phenyl | S-(CH₂)₃- | CH₂ | 6-methoxy | |
| 202 | Me | 2,5-Di-methyl-furanyl-3- | S-(CH₂)₃- | CH₂ | 8-fluor | |
| 203 | Propyl | 3-Jod-phenyl | COO-(CH₂)₄- | CH₂ | 8-trifluormethoxy | |
| 204 | isoPropyl | 4-Methoxyphenyl | S-(CH₂)₃- | CH₂ | 7-sulfonamido | |
| 205 | Me | N-Methyl-2-Pyrrolyl- | S-(CH₂)₃- | CH₂ | 7-cyano | |
| 206 | Ethyl | Pyridin-3-yl- | S-CH₂-C(CH₃)=CH-CH₂- | O | 7-tert-butyl | |
| 207 | cycPropyl | 3-Benzthienyl- | S-(CH₂)₃- | CH₂ | 8-fluor | |
| 208 | Me | Pyridin-3-yl- | S-(CH₂)₃- | CH₂ | 7-sulfonamido | |
| 209 | Phenyl | 4-Imidazolyl- | S-(CH₂)₃- | CH₂ | H | |
| 210 | Propyl | Phenyl | S-(CH₂)₃- | O | 7-carboxamido | |
| 211 | Me | Phenyl | S-(CH₂)₇- | O | 7-tert-butyl | |
| 212 | Me | 4-Methoxyphenyl | S-(CH₂)₃- | O | 7-cyano | |
| 213 | Me | 3-Br-Pyridin-5-yl- | S-(CH₂)₃- | CH₂ | 7-tert-butyl | |
| 214 | Hexyl | 4-Methoxyphenyl | S-(CH₂)₃- | CH₂ | 6-methoxy | |
| 215 | isoPropyl | Methylamino | S-(CH₂)₃- | CH₂ | 6-methoxy | |
| 216 | Me | 2,5-Di-methyl-furanyl-3- | S-(CH₂)₃- | CH₂ | 6-methoxy | |
| 217 | Me | N-Methyl-2-Pyrrolyl- | S-(CH₂)₃- | CH₂ | 7-tert-butyl | |
| 218 | Me | Phenyl | O-(CH₂)₃- | CH₂ | 7-sulfonamido | |
| 219 | Me | Pyridin-3-yl- | S-(CH₂)₃- | CH₂ | 7-methoxy | 8-methoxy |
| 220 | Ethyl | N-Methyl-2-Pyrrolyl- | (CH₂)₄- | O | 9-methyl | |
| 221 | isoPropyl | Tetrazolyl- | S-(CH₂)₃- | CH₂ | 6-methoxy | |
| 222 | Me | 4-Methylthiazol-5-yl | S-(CH₂)₃- | CH₂ | 7-sulfonamido | |
| 223 | Me | N-Methyl-2-Pyrrolyl- | S-(CH₂)₃- | O | 7-cyano | |
| 224 | Me | 5-Methyl imidazol-4-yl- | S-(CH₂)₃- | CH₂ | 7-tert-butyl | |
| 225 | Me | Phenyl | S-(CH₂)₇- | CH₂ | 7-tert-butyl | |
| 226 | Ethyl | 2-Thienyl | CO-(CH₂)₃- | O | H | |
| 227 | Me | Methylamino | S-(CH₂)₃- | CH₂ | 7-methoxy | 8-methoxy |
| 228 | isoPropyl | 3-Benzthienyl- | S-(CH₂)₃- | O | 8-trifluormethoxy | |
| 229 | Me | 4-Methylthiazol-5-yl | S-(CH₂)₃- | O | 7-cyano | |
| 230 | Propyl | Phenyl | S-(CH₂)₃- | CH₂ | 7-carboxamido | |
| 231 | Me | Phenyl | S-(CH₂)₃- | CH₂ | H | |
| 232 | isoPropyl | Phenyl | S-(CH₂)₃- | CH₂ | 7-sulfonamido | |
| 233 | Me | 3-Jod-phenyl | O-(CH₂)₃- | O | 7-nitro | |
| 234 | Me | 4-Methylthiazol-5-yl | S-(CH₂)₃- | CH₂ | 7-nitro | |
| 235 | Me | Phenyl | S-(CH₂)₃- | O | 7-cyano | |
| 236 | Me | 2-Thienyl | S-(CH₂)₃- | CH₂ | H | |
| 237 | Et | Amino | S-(CH₂)₃- | CH₂ | 7-tert-butyl | |
| 238 | Me | 4-Methylthiazol-5-yl | S-(CH₂)₃- | CH₂ | 6-methoxy | |
| 239 | Me | N-Methyl-2-Pyrrolyl- | S-(CH₂)₃- | CH₂ | 6-methoxy | |
| 240 | cycPropyl | Cyclohexyl- | S-(CH₂)₃- | CH₂ | 7-tert-butyl | |
| 241 | Ethyl | Phenyl | CO-(CH₂)₃- | CH₂ | H | |
| 242 | Me | Pyridin-3-yl- | S-(CH₂)₃- | CH₂ | H | |
| 243 | Me | 4-Methoxyphenyl | S-(CH₂)₃- | O | H | |
| 244 | Me | N-Propyl-tetrazolyl- | S-(CH₂)₃- | CH₂ | H | |
| 245 | Me | 2-Pyrazinyl- | S-(CH₂)₃- | CH₂ | H | |
| 246 | Ethyl | 4-Methylthiazol-5-yl | S-CH₂-C(CH₃)=CH-CH₂- O | | 7-tert-butyl | |
| 247 | Me | 2-Thienyl | S-(CH₂)₃- | CH₂ | 7-nitro | |
| 248 | Me | 4-Methylthiazol-5-yl | S-(CH₂)₃- | CH₂ | 8-fluor | |
| 249 | Phenyl | 3-Benzthienyl- | S-(CH₂)₃- | O | H | |
| 250 | Me | 3-Jod-phenyl | S-(CH₂)₃- | O | 7-tert-butyl | |
| 251 | Me | N-Methyl-2-Pyrrolyl- | S-(CH₂)₃- | CH₂ | 8-fluor | |
| 252 | Et | Pyridin-4-yl- | S-(CH₂)₃- | CH₂ | 7-tert-butyl | |
| 253 | Phenyl | Carboxamido | S-(CH₂)₃- | CH₂ | 8-fluor | |
| 254 | Ethyl | Phenyl | S-(CH₂)₃- | O | 7-nitro | |
| 255 | Me | 3-Pyrrolyl | S-(CH₂)₃- | CH₂ | 7-carboxamido | |
| 256 | isoPropyl | 3-Pyrrolyl | S-(CH₂)₃- | O | 8-trifluormethoxy | |
| 257 | Me | Amino | S-(CH₂)₃- | CH₂ | 7-methoxy | 8-methoxy |
| 258 | Propyl | Phenyl | COO-(CH₂)₄- | O | 7-methyl | |
| 259 | Me | Oxadiazol-2-yl | S-(CH₂)₃- | O | 7-methoxy | 8-methoxy |
| 260 | Me | 2-Aminothiazol-4yl- | S-(CH₂)₃- | CH₂ | 8-fluor | |
| 261 | Phenyl | Amino | S-(CH₂)₃- | CH₂ | H | |
| 262 | Me | Phenyl | S-(CH₂)₁₀- | O | 7-methyl | |
| 263 | Me | Phenyl | S-(CH₂)₃- | CH₂ | 8-fluor | |
| 264 | Me | Pyridin-4-yl- | S-(CH₂)₃- | CH₂ | H | |
| 265 | Ethyl | 4-Methylthiazol-5-yl | S-(CH₂)₆- | O | 7-cyano | |
| 266 | Hexyl | Pyridin-4-yl- | S-(CH₂)₃- | CH₂ | 6-methoxy | |
| 267 | Me | 5-Methyl imidazol-4-yl- | S-(CH₂)₃- | O | H | |
| 268 | butyl | Cyano | S-(CH₂)₃- | CH₂ | 7-methoxy | 8-methoxy |
| 269 | Me | 4-Methylthiazol-5-yl | S-(CH₂)₃- | CH₂ | 7-methoxy | 8-methoxy |
| 270 | Me | 2-Pyrazinyl- | (CH₂)₄- | CH₂ | 7-methyl | |
| 271 | Me | 4-Methylthiazol-5-yl | S-(CH₂)₃- | CH₂ | H | |
| 272 | Me | Cyclohexyl- | S-(CH₂)₃- | CH₂ | 6-methoxy | |
| 273 | Me | 4-Methoxyphenyl | S-(CH₂)₃- | O | 7-methoxy | 8-methoxy |
| 274 | Me | 2-Thienyl | S-(CH₂)₇- | O | 8-trifluormethoxy | |
| 275 | Phenyl | Cyano | S-(CH₂)₃- | O | H | |
| 276 | Me | Pyridin-3-yl- | S-(CH₂)₃- | CH₂ | 7-cyano | |
| 277 | Me | Phenyl | (CH₂)₄- | CH₂ | H | |
| 278 | butyl | 3-Pyrrolyl | S-(CH₂)₃- | CH₂ | 7-methoxy | 8-methoxy |
| 279 | Me | Phenyl | S-(CH₂)₇- | O | 7-tert-butyl | |
| 280 | Me | 4-Methylthiazol-5-yl | S-(CH₂)₃- | CH₂ | 7-cyano | |
| 281 | Me | 2-Thienyl | S-CH₂-C(=CH₂)-CH₂ | O | 8-fluor | |
| 282 | Me | 4-Methoxyphenyl | S-(CH₂)₃- | CH₂ | 7-methoxy | 8-methoxy |
| 283 | Me | 5-Methyl imidazol-4-yl- | S-(CH₂)₃- | CH₂ | H | |
| 284 | Me | 4-Methylthiazol-5-yl | S-(CH₂)₃- | O | 7-cyano | |
| 285 | Me | 2-Pyrazinyl- | S-(CH₂)₃- | CH₂ | 7-cyano | |
| 286 | Me | 3-Jod-phenyl | S-(CH₂)₃- | O | 7-nitro | |
| 287 | Me | 2-Thienyl | S-(CH₂)₈- | CH₂ | 6-methoxy | |
| 288 | isoPropyl | Oxadiazol-2-yl | S-(CH₂)₃- | CH₂ | 6-methoxy | |
| 289 | Me | 3-Br-Pyridin-5-yl- | S-(CH₂)₃- | CH₂ | 6-methoxy | |
| 290 | Me | Pyridin-3-yl- | S-(CH₂)₃- | CH₂ | 7-nitro | |
| 291 | Me | 4-Methoxyphenyl | S-(CH₂)₃- | CH₂ | H | |
| 292 | Ethyl | 4-Methylthiazol-5-yl | S-(CH₂)₆- | CH₂ | 7-cyano | |
| 293 | Me | 2-Pyrazinyl- | S-(CH₂)₃- | CH₂ | 7-methoxy | 8-methoxy |
| 294 | Me | 3-Pyrrolyl | S-(CH₂)₃- | CH₂ | H | |
| 295 | Me | 4-Methoxyphenyl | O-(CH₂)₃- | CH₂ | H | |
| 296 | Me | 3-Jod-phenyl | S-(CH₂)₃- | O | 7-cyano | |
| 297 | Ethyl | Pyridin-3-yl- | S-CH₂-C(CH₃)=CH-CH₂- | CH₂ | 7-tert-butyl | |

| | | | | | | |
|---|---|---|---|---|---|---|
| * Soweit für R⁷ keine Bedeutung angegeben wird, steht R⁷ für Wasserstoff. In dieser und den nachfolgenden Tabellen werden die folgenden Abkürzungen verwendet: Me = Methyl Et = Ethyl cycPropyl = Cyclopropyl | | | | | | |

In prinzipiell analoger Weise können hergestellt werden:

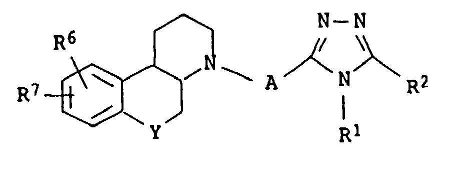

**Tabelle 2:**

| Bsp | R¹ | R² | A | Y | R⁶ | R⁷* |
|---|---|---|---|---|---|---|
| 298 | Me | N-Methyl-2-Pyrrolyl- | (CH₂)₄- | CH₂ | 10-methyl | |
| 299 | Ethyl | 2-Aminothiazol-4yl- | S-(CH₂)₃- | O | H | |
| 300 | Me | 3-Jod-phenyl | S-(CH₂)₃- | O | 8-nitro | |
| 301 | Me | Amino | S-(CH₂)₃- | CH₂ | 8-tert-butyl | |
| 302 | Me | 5-Methyl imidazol-4-yl- | S-(CH₂)₃- | CH₂ | H | |
| 303 | Me | Cyclohexyl- | S-(CH₂)₃- | CH₂ | 8-tert-butyl | |
| 304 | Me | 4-Methoxyphenyl | S-(CH₂)₃- | O | H | |
| 305 | Ethyl | Pyridin-3-yl- | S-(CH₂)₆- | O | 8-tert-butyl | |
| 306 | Me | 3-Jod-phenyl | (CH₂)₄- | O | 8-tert-butyl | |
| 307 | Me | 5-Methyl imidazol-4-yl- | S-(CH₂)₃- | CH₂ | 8-tert-butyl | |
| 308 | Me | 2-Thienyl | (CH₂)₄- | O | H | |
| 309 | Me | 2-Me-4-Oxazolyl- | S-(CH₂)₃- | CH₂ | 9-methyl | |
| 310 | Me | 2,5-Di-methyl-furanyl-3- | S-(CH₂)₃- | CH₂ | 7-methoxy | |
| 311 | Me | 4-Methylthiazol-5-yl | S-(CH₂)₃- | O | 8-cyano | |
| 312 | Propyl | 4-Methoxyphenyl | COO-(CH₂)₃- | CH₂ | 8-sulfonamido | |
| 313 | Propyl | N-Methyl-2-Pyrrolyl- | CONH-(CH₂)₄- | CH₂ | 9-trifluormethoxy | |
| 314 | Me | 2-Thienyl | S-(CH₂)₇- | CH₂ | 9-trifluormethoxy | |
| 315 | Me | Pyridin-3-yl- | S-(CH₂)₃- | CH₂ | 9-methyl | |
| 316 | Me | N-Propyl-tetrazolyl- | S-(CH₂)₃- | CH₂ | 9-trifluormethoxy | |
| 317 | Me | 3-Br-Pyridin-5-yl- | S-(CH₂)₃- | CH₂ | 8-tert-butyl | |
| 318 | Me | 3-Cyano-phenyl | S-(CH₂)₃- | CH₂ | 7-methoxy | |
| 319 | Phenyl | Amino | S-(CH₂)₃- | O | H | |
| 320 | Butyl | 2,5-Di-methyl-furanyl-3- | S-(CH₂)₃- | CH₂ | 8-methoxy | 9-methoxy |
| 321 | Phenyl | 3-Benzthienyl- | S-(CH₂)₃- | O | H | |
| 322 | Me | N-Methyl-2-Pyrrolyl- | S-(CH₂)₃- | CH₂ | 8-tert-butyl | |
| 323 | Propyl | 3-cyano-phenyl | CONH-(CH₂)₅- | CH₂ | 8-methyl | |
| 324 | Me | 3-Jod-phenyl | S-(CH₂)₃- | CH₂ | 8-sulfonamido | |
| 325 | Me | Phenyl | O-(CH₂)₃- | CH₂ | 8-sulfonamido | |
| 326 | Me | 4-Methylthiazol-5-yl | (CH₂)₄- | CH₂ | 10-methyl | |
| 327 | Me | 3-Pyrrolyl | S-(CH₂)₃- | CH₂ | 7-methoxy | |
| 328 | Butyl | 3-Cyano-phenyl | S-(CH₂)₃- | CH₂ | 8-methoxy | 9-methoxy |
| 329 | Ethyl | 4-Methylthiazol-5-yl | S-(CH₂)₆- | O | 8-cyano | |
| 330 | Me | Pyridin-4-yl- | S-(CH₂)₃- | O | H | |
| 331 | Butyl | 2-Aminothiazol-4yl- | S-(CH₂)₃- | CH₂ | 8-methoxy | 9-methoxy |
| 332 | Propyl | 4-Imidazolyl- | S-(CH₂)₃- | CH2 | 8-carboxamido | |
| 333 | isoPropyl | Tetrazolyl- | S-(CH₂)₃- | CH₂ | 7-methoxy | |
| 334 | Me | Pyridin-3-yl- | S-(CH₂)₃- | O | 8-cyano | |
| 335 | Me | N-Methyl-2-Pyrrolyl- | S-(CH₂)₃- | O | 8-nitro | |
| 336 | Et | Amino | S-(CH₂)₃- | CH₂ | H | |
| 337 | Phenyl | Methylamino | S-(CH₂)₃- | CH₂ | 9-methyl | |
| 338 | Me | 4-Methylthiazol-5-yl | (CH₂)₄- | O | 10-methyl | |
| 339 | Me | 4-Methylthiazol-5-yl | S-(CH₂)₃- | CH₂ | 8-sulfonamido | |
| 340 | Me | Methylamino | S-(CH₂)₃- | CH₂ | 8-tert-butyl | |
| 341 | Et | 3-Cyano-phenyl | S-(CH₂)₃- | CH₂ | H | |
| 342 | Me | 4-Methylthiazol-5-yl | S-(CH₂)₃- | O | 8-nitro | |
| 343 | Me | 3-Jod-phenyl | (CH₂)₄- | CH₂ | 8-tert-butyl | |
| 344 | Me | 4-Methylthiazol-5-yl | S-(CH₂)₃- | CH₂ | H | |
| 345 | Me | 3-Br-Pyridin-5-yl- | S-(CH₂)₃- | CH₂ | H | |
| 346 | Propyl | Pyridin-4-yl- | S-(CH₂)₃- | CH₂ | 8-carboxamido | |
| 347 | Me | 4-Methoxyphenyl | S-(CH₂)₃- | CH₂ | 8-cyano | |
| 348 | isoPropyl | 3-Benzthienyl- | S-(CH₂)₃- | CH₂ | 9-trifluormethoxy | |
| 349 | Butyl | 3-Benzthienyl- | S-(CH₂)₃- | CH₂ | 8-methoxy | 9-methoxy |
| 350 | Ethyl | Pyridin-3-yl- | (CH₂)₄- | O | H | |
| 351 | Propyl | 2-Aminothiazol-4yl- | S-(CH₂)₃- | CH₂ | 8-carboxamido | |
| 352 | Me | Cyclohexyl- | S-(CH₂)₃- | CH₂ | 9-methyl | |
| 353 | Ethyl | Phenyl | S-(CH₂)₃- | O | 8-nitro | |
| 354 | Me | 4-Methoxyphenyl | S-(CH₂)₃- | O | 8-methoxy | 9-methoxy |
| 355 | Propyl | Phenyl | COO-(CH₂)₄- | O | 8-methyl | |
| 356 | Me | 2,5-Di-methyl-furanyl-3- | S-(CH₂)₃- | CH₂ | 9-methyl | |
| 357 | Me | 3-Jod-phenyl | S-(CH₂)₃- | CH₂ | H | |
| 358 | Me | Phenyl | S-(CH₂)₃- | CH₂ | H | |
| 359 | isoPropyl | Oxadiazol-2-yl | S-(CH₂)₃- | CH₂ | 7-methoxy | |
| 360 | Me | Phenyl | S-(CH₂)₇- | O | 8-tert-butyl | |
| 361 | Propyl | 2-Pyrazinyl- | COO-(CH₂)₄- | CH₂ | 9-trifluormethoxy | |
| 362 | Me | 3-Pyrrolyl | S-(CH₂)₃- | CH₂ | 8-carboxamido | |
| 363 | Propyl | Phenyl | S-(CH₂)₃- | CH₂ | 8-carboxamido | |
| 364 | Me | 2-Pyrazinyl- | O-(CH₂)₃- | CH₂ | H | |
| 365 | Me | Phenyl | S-(CH₂)₇- | O | 8-tert-butyl | |
| 366 | Me | Pyridin-3-yl- | S-(CH₂)₃- | CH₂ | 8-sulfonamido | |
| 367 | Me | 3-Jod-phenyl | S-(CH₂)₃- | CH₂ | 8-cyano | |
| 368 | Me | 2-Thienyl | S-(CH₂)₃- | CH₂ | 9-methyl | |
| 369 | isoPropyl | Methylamino | S-(CH₂)₃- | CH₂ | 7-methoxy | |
| 370 | Ethyl | 3-Jod-phenyl | S-(CH₂)₃- | CH₂ | 8-methoxy | 9-methoxy |
| 371 | Ethyl | Phenyl | CO-(CH₂)₃- | O | H | |
| 372 | Phenyl | 4-Imidazolyl- | S-(CH₂)₃- | O | H | |
| 373 | Ethyl | 4-Methylthiazol-5-yl | S-(CH₂)₆- | CH₂ | 8-cyano | |
| 374 | Me | 3-Jod-phenyl | S-(CH₂)₃- | CH₂ | 7-methoxy | |
| 375 | Hexyl | 4-Imidazolyl- | S-(CH₂)₃- | CH₂ | 7-methoxy | |
| 376 | Me | Phenyl | S-(CH₂)₃- | CH₂ | 9-trifluormethoxy | |
| 377 | Me | 3-Jod-phenyl | O-(CH₂)₃- | O | 8-nitro | |
| 378 | Me | 5-Methyl imidazol-4-yl- | S-(CH₂)₃- | CH₂ | 7-methoxy | |
| 379 | Ethyl | Pyridin-3-yl- | S-CH₂-C(CH₃)=CH-CH₂- | O | 8-tert-butyl | |
| 380 | Me | N-Methyl-2-Pyrrolyl- | S-(CH₂)₃- | CH₂ | 7-methoxy | |
| 381 | Ethyl | Phenyl | S-CH₂-C(CH₃)=CH-CH₂- | CH₂ | 8-tert-butyl | |
| 382 | Me | Pyridin-3-yl- | S-(CH₂)₈- | O | 9-trifluormethoxy | |
| 383 | Me | 2-Thienyl | S-(CH₂)₃- | O | 8-nitro | |
| 384 | Me | 2-Pyrazinyl- | (CH₂)₄- | O | 8-methyl | |
| 385 | Butyl | 3-Br-Pyridin-5-yl- | S-(CH₂)₃- | CH₂ | 8-methoxy | 9-methoxy |
| 386 | Me | 2-Pyrazinyl- | S-(CH₂)₃- | CH₂ | 8-methoxy | 9-methoxy |
| 387 | Me | Phenyl | S-(CH₂)₃- | O | 8-cyano | |
| 388 | Me | 4-Methoxyphenyl | S-(CH₂)₃- | O | 8-nitro | |
| 389 | Me | Cyclohexyl- | S-(CH₂)₃- | CH₂ | 7-methoxy | |
| 390 | Me | Phenyl | CO-CH₂-C(=CH₂)-CH₂ | O | 8-tert-butyl | |
| 391 | Me | N-Methyl-2-Pyrrolyl- | (CH₂)₄- | O | 10-methyl | |
| 392 | Me | Oxadiazol-2-yl | S-(CH₂)₃- | CH₂ | 9-methyl | |
| 393 | Me | 2-Pyrazinyl- | S-(CH₂)₃- | CH₂ | H | |
| 394 | Ethyl | Phenyl | S-(CH₂)₃- | CH₂ | 8-nitro | |
| 395 | Me | Pyridin-3-yl- | S-(CH₂)₃- | CH₂ | 8-nitro | |
| 396 | Me | 2-Me-4-Oxazolyl- | S-(CH₂)₃- | CH₂ | 7-methoxy | |
| 397 | Me | Pyridin-3-yl- | S-(CH₂)₃- | CH₂ | 8-tert-butyl | |
| 398 | Ethyl | Phenyl | CO-(CH₂)₃- | CH₂ | H | |
| 399 | Me | 3-Cyano-phenyl | S-(CH₂)₃- | O | H | |
| 400 | Me | Pyridin-3-yl- | (CH₂)₄- | O | H | |
| 401 | Me | 4-Jod-phenyl | S-(CH₂)₃- | CH₂ | 8-tert-butyl | |
| 402 | Butyl | 3-Pyrrolyl | S-(CH₂)₃- | CH₂ | 8-methoxy | 9-methoxy |
| 403 | Me | N-Methyl-2-Pyrrolyl- | S-(CH₂)₃- | O | 8-cyano | |
| 404 | Me | 2-Me-4-Oxazolyl- | S-(CH₂)₃- | CH₂ | 8-tert-butyl | |
| 405 | isoButyl | Methylamino | S-(CH₂)₃- | O | H | |
| 406 | Me | 2-Thienyl | S-(CH₂)₃- | O | 8-nitro | |
| 407 | Me | 2-Thienyl | S-(CH₂)₇- | O | 9-trifluormethoxy | |
| 408 | Me | 4-Methylthiazol-5-yl | S-(CH₂)₃- | O | 8-cyano | |
| 409 | Me | 2-Me-4-Oxazolyl- | S-(CH₂)₃- | O | 8-tert-butyl | |
| 410 | Me | 2-Thienyl | S-CH₂-C(=CH₂)-CH₂ | CH₂ | 9-fluor | |
| 411 | Me | N-Methyl-2-Pyrrolyl- | S-(CH₂)₃- | O | 8-cyano | |
| 412 | Me | Phenyl | S-(CH₂)₃- | CH₂ | 9-methyl | |
| 413 | Me | 3-Jod-phenyl | S-(₂)₃- | CH₂ | 9-methyl | |
| 414 | Me | Pyridin-3-yl- | S-(CH₂)₃- | O | 8-tert-butyl | |
| 415 | Ethyl | 2-Thienyl | (CH₂)₄- | O | H | |
| 416 | Me | Tetrazolyl- | S-(CH₂)₃- | CH₂ | 9-methyl | |
| 417 | Me | 2-Thienyl | S-(CH₂)₈- | CH₂ | 7-methoxy | |
| 418 | Me | Pyridin-4-yl- | S-(CH₂)₃- | CH₂ | 9-trifluormethoxy | |
| 419 | Me | Phenyl | S-CH₂-C(=CH₂)-CH₂ | CH₂ | 9-brom | |
| 420 | Me | Phenyl | S-(CH₂)₁₀- | O | 8-methyl | |
| 421 | Me | Oxadiazol-2-yl | S-(CH₂)₃- | CH₂ | H | |
| 422 | Me | Oxadiazol-2-yl | S-(CH₂)₃- | O | 8-methoxy | 9-methoxy |
| 423 | Butyl | 4-Imidazolyl- | S-(CH₂)₃- | CH₂ | 8-methoxy | 9-methoxy |
| 424 | isoPropyl | 3-Pyrrolyl | S-(CH₂)₃- | O | 9-trifluormethoxy | |
| 425 | isoPropyl | 3-Cyano-phenyl | S-(CH₂)₃- | CH₂ | 9-trifluormethoxy | |
| 426 | Butyl | N-Propyl-tetrazolyl- | S-(CH₂)₃- | CH₂ | 8-methoxy | 9-methoxy |
| 427 | Me | Pyridin-3-yl- | S-(CH₂)₈- | CH₂ | 9-trifluormethoxy | |
| 428 | cycPropyl | 4-Methoxyphenyl | S-(CH₂)₃- | CH₂ | 9-methyl | |
| 429 | Me | Phenyl | CO-CH₂-C(=CH₂)-CH₂ | CH₂ | 8-tert-butyl | |
| 430 | Me | 3-Br-Pyridin-5-yl- | S-(CH₂)₃- | CH₂ | 9-methyl | |
| 431 | Me | 2-Pyrazinyl- | (CH₂)₄- | CH₂ | 8-methyl | |
| 432 | Me | Phenyl | CO-CH₂-C(=CH₂)-CH₂ | O | 8-tert-butyl | |
| 433 | Me | N-Methyl-2-Pyrrolyl- | S-(CH₂)₃- | CH₂ | 8-cyano | |
| 434 | Me | 2-Thienyl | S-(CH₂)₃- | CH₂ | 8-methoxy | 9-methoxy |
| 435 | Ethyl | Pyridin-3-yl- | S-CH₂-C(CH₃)=CH-CH₂- | CH₂ | 8-tert-butyl | |
| 436 | Me | Methylamino | S-(CH₂)₃- | CH₂ | 8-methoxy | 9-methoxy |
| 437 | Me | 2-Me-4-Oxazolyl- | S-(CH₂)₃- | CH₂ | 8-methoxy | 9-methoxy |
| 438 | isoPropyl | Phenyl | S-(CH₂)₃- | O | 8-sulfonamido | |
| 439 | Ethyl | Phenyl | S-CH₂-C(CH₃)=CH-CH₂- | O | 8-tert-butyl | |
| 440 | Me | 3-Br-Pyridin-5-yl- | S-(CH₂)₃- | CH₂ | 7-methoxy | |
| 441 | Me | Cyano | S-(CH₂)₃- | CH₂ | H | |
| 442 | Me | 3-Br-Pyridin-5-yl- | S-(CH₂)₃- | O | H | |
| 443 | Me | N-Methyl-2-Pyrrolyl- | S-(CH₂)₃- | CH₂ | 8-nitro | |
| 444 | Me | N-Propyl-tetrazolyl- | S-(CH₂)₃- | CH₂ | 8-tert-butyl | |
| 445 | Me | 4-Methoxyphenyl | S-(CH₂)₃- | CH₂ | 8-methoxy | 9-methoxy |
| 446 | Me | 2-Pyrazinyl- | S-(CH₂)₃- | CH₂ | 8-sulfonamido | |
| 447 | Me | Phenyl | S-(CH₂)₃- | CH₂ | 8-methoxy | 9-methoxy |
| 448 | Me | 4-Methoxyphenyl | (CH₂)₄- | CH₂ | 10-fluor | |
| 449 | Me | 2-Thienyl | S-(CH₂)₃- | CH₂ | 8-sulfonamido | |
| 450 | isoPropyl | 5-Methyl imidazol-4-yl- | S-(CH₂)₃- | CH₂ | 9-trifluormethoxy | |
| 451 | Ethyl | 3-Jod-phenyl | (CH₂)₄- | O | 8-tert-butyl | |
| 452 | Me | 2-Pyrazinyl- | S-(CH₂)₃- | CH₂ | 8-cyano | |
| 453 | isoPropyl | 2-Aminothiazol-4yl- | S-(CH₂)₃- | CH₂ | 9-trifuormethoxy | |
| 454 | cycPropyl | 3-Cyano-phenyl | S-(CH₂)₃- | CH₂ | 9-methyl | |
| 455 | isoPropyl | 3-Pyrrolyl | S-(CH₂)₃- | CH₂ | 9-trifluormethoxy | |
| 456 | Me | 2-Aminothiazol-4yl- | S-(CH₂)₃- | CH₂ | 7-methoxy | |
| 457 | Me | Pyridin-3-yl- | S-(CH₂)₃- | CH₂ | 7-methoxy | |
| 458 | Me | 2-Aminothiazol-4yl- | S-(CH₂)₃- | CH₂ | H | |
| 459 | Me | Phenyl | O-(CH₂)₄- | O | 9-iod | |
| 460 | Me | 2-Thienyl | S-(CH₂)₃- | O | 8-cyano | |
| 461 | Ethyl | Pyridin-3-yl- | S-CH₂-CH=CH-CH₂- | O | 9-fluor | |
| 462 | Propyl | 4-Methylthiazol-5-yl | CONH-(CH₂)₄- | CH₂ | 8-methyl | |
| 463 | Phenyl | 4-Imidazolyl- | S-(CH₂)₃- | CH₂ | H | |
| 464 | Hexyl | 3-Benzthienyl- | S-(CH₂)₃- | CH₂ | 7-methoxy | |
| 465 | Ethyl | Pyridin-3-yl- | S-(CH₂)₆- | CH₂ | 8-tert-butyl | |
| 466 | Me | 3-Pyrrolyl | S-(CH₂)₃- | O | H | |
| 467 | cycPropyl | 2-Thienyl | S-(CH₂)₃- | CH₂ | H | |
| 468 | Ethyl | Phenyl | S-CH₂-C(CH₃)=CH-CH₂- | O | 8-sulfonamido | |
| 469 | Me | 2-Aminothiazol-4yl- | S-(CH₂)₃- | CH₂ | 8-tert-butyl | |
| 470 | isoPropyl | 3-Benzthienyl- | S-(CH₂)₃- | O | 9-trifuormethoxy | |
| 471 | Me | Pyridin-3-yl- | S-(CH₂)₃- | O | 8-nitro | |
| 472 | Ethyl | 2-Thienyl | CO-(CH₂)₃- | CH₂ | H | |
| 473 | Me | N-Methyl-2-Pyrrolyl- | S-(CH₂)₃- | CH₂ | H | |
| 474 | Propyl | Phenyl | CONH-(CH₂)₅- | O | 8-methyl | |
| 475 | isoPropyl | 5-Methyl imidazol-4-yl- | S-(CH₂)₃- | O | 9-trifluormethoxy | |
| 476 | Me | 4-Methoxyphenyl | S-(CH2)3- | CH2 | 8-tert-butyl | |
| 477 | Me | N-Methyl-2-Pyrrolyl- | S-(CH₂)₃- | CH₂ | 8-methoxy | 9-methoxy |
| 478 | cycPropyl | N-Propyl-tetrazolyl- | S-(CH₂)₃- | CH₂ | 9-methyl | |
| 479 | Et | 2,5-Di-methyl-furanyl-3- | S-(CH₂)₃- | CH₂ | H | |
| 480 | isoButyl | Oxadiazol-2-yl | S-(CH₂)₃- | O | 8-tert-butyl | |
| 481 | Hexyl | N-Propyl-tetrazolyl- | S-(CH₂)₃- | CH₂ | 7-methoxy | |
| 482 | Propyl | 2-Thienyl | COO-(CH₂)₄- | CH₂ | 7-methoxy | |
| 483 | Phenyl | Cyano | S-(CH₂)₃- | O | H | |
| 484 | Me | 2-Thienyl | S-CH₂-C(=CH₂)-CH₂ | O | 9-fluor | |
| 485 | Me | Pyridin-4-yl- | S-(CH₂)₃- | CH₂ | 8-tert-butyl | |
| 486 | Me | 2-Pyrazinyl- | S-(CH₂)₃- | O | 8-cyano | |
| 487 | Butyl | 5-Methyl imidazol-4-yl- | S-(CH₂)₃- | CH₂ | 8-methoxy | 9-methoxy |
| 488 | Ethyl | 2-Thienyl | CO-(CH₂)₃- | O | H | |
| 489 | Me | Oxadiazol-2-yl | S-(CH₂)₃- | CH₂ | 8-tert-butyl | |
| 490 | isoPropyl | Cyano | S-(CH₂)₃- | CH₂ | 7-methoxy | |
| 491 | Me | Amino | S-(CH₂)₃- | CH₂ | 8-methoxy | 9-methoxy |
| 492 | Me | N-Methyl-2-Pyrrolyl- | S-CH₂-CH=CH-CH₂- | CH₂ | 9-fluor | |
| 493 | Me | Phenyl | S-(CH₂)₇- | CH₂ | 8-tert-butyl | |
| 494 | Me | 2-Pyrazinyl- | O-(CH₂)₃- | O | H | |
| 495 | Me | Phenyl | S-(CH₂)₁₀- | CH₂ | 8-methyl | |
| 496 | Butyl | Pyridin-4-yl- | S-(CH₂)3- | CH₂ | 8-methoxy | 9-methoxy |
| 497 | Ethyl | 4-Methylthiazol-5-yl | S-(CH₂)₆- | O | 8-cyano | |
| 498 | Me | 4-Methoxyphenyl | S-(CH₂)₃- | O | 8-cyano | |
| 499 | Me | 3-Pyrrolyl | S-(CH₂)3- | CH₂ | 9-methyl | |
| 500 | isoPropyl | Phenyl | S-(CH₂)₃- | CH₂ | 8-sulfonamido | |
| 501 | Me | Tetrazolyl- | S-(CH₂)3- | CH₂ | 8-methoxy | 9-methoxy |
| 502 | Phenyl | 2-Pyrazinyl- | S-(CH₂)3- | CH₂ | 8-tert-butyl | |
| 503 | isoPropyl | 4-Methoxyphenyl | S-(CH₂)₃- | CH₂ | 8-sulfonamido | |
| 504 | Phenyl | Carboxamido | S-(CH₂)₃- | CH₂ | 9-methyl | |
| 505 | Me | 4-Methylthiazol-5-yl | S-(CH₂)₃- | O | H | |
| 506 | Me | 3-Cyano-phenyl | S-(CH₂)₃- | CH₂ | 8-tert-butyl | |
| 507 | Pentyl | Phenyl | CH₂-CH₂-CH=CH-CH₂- | O | H | |
| 508 | Me | N-Methyl-2-Pyrrolyl- | S-(CH₂)₃- | CH₂ | 9-methyl | |
| 509 | Me | Pyridin-3-yl- | S-(CH₂)₃- | CH₂ | 8-methoxy | 9-methoxy |
| 510 | Me | 4-Methoxyphenyl | O-(CH₂)₃- | CH₂ | H | |
| 511 | Me | 2-Pyrazinyl- | S-(CH₂)₃- | CH₂ | 9-methyl | |
| 512 | Phenyl | 3-Benzthienyl- | S-(CH₂)₃- | CH₂ | 8-tert-butyl | |
| 513 | Me | Phenyl | O-(CH₂)₄- | CH₂ | 8-tert-butyl | |
| 514 | Me | 3-Jod-phenyl | O-(CH₂)₃- | O | H | |
| 515 | Me | 3-Thienyl | S-(CH₂)₃- | CH₂ | 8-tert-butyl | |
| 516 | cycPropyl | Pyridin-3-yl- | S-(CH₂)₃- | CH₂ | H | |
| 517 | Me | Pyridin-3-yl- | S-(CH₂)₃- | CH₂ | 8-cyano | |
| 518 | Me | 4-Methylthiazol-5-yl | S-(CH₂)₃- | CH₂ | 8-nitro | |
| 519 | Me | Phenyl | S-CH₂-C(=CH₂)-CH₂ | O | 9-brom | |
| 520 | Me | Pyridin-3-yl- | (CH₂)4- | CH₂ | H | |
| 521 | Me | 2-Thienyl | S-(CH₂)₃- | CH₂ | 7-methoxy | |
| 522 | Propyl | Phenyl | CONH-(CH₂)5- | CH₂ | 8-methyl | |
| 523 | Me | N-Propyl-tetrazolyl- | S-(CH₂)₃- | O | H | |
| 524 | Ethyl | Phenyl | S-(CH₂)₃- | O | 8-nitro | |
| 525 | Me | Phenyl | (CH₂)₄- | CH₂ | H | |
| 526 | Propyl | 3-Cyano-phenyl | S-(CH₂)₃- | CH₂ | 8-carboxamido | |
| 527 | Me | Oxadiazol-2-yl | S-(CH₂)₃- | CH₂ | 8-methoxy | 9-methoxy |
| 528 | Me | 4-Methoxyphenyl | S-(CH₂)₃- | CH₂ | 8-nitro | |
| 529 | Me | Phenyl | (CH₂)₄- | O | H | |
| 530 | isoPropyl | 3-Br-Pyridin-5-yl- | S-(CH₂)₃- | CH₂ | 9-trifluormethoxy | |
| 531 | Ethyl | 4-Methylthiazol-5-yl | S-CH₂-C(=CH₂)-CH₂ | CH₂ | 8-sulfonamido | |
| 532 | Me | Tetrazolyl- | S-(CH₂)₃- | CH₂ | H | |
| 533 | Me | 4-Methylthiazol-5-yl | S-(CH₂)₃- | CH₂ | 8-methoxy | 9-methoxy |
| 534 | Propyl | Phenyl | COO-(CH₂)₄- | CH₂ | 8-methyl | |
| 535 | Propyl | 3-Br-Pyridin-5-yl- | S-(CH₂)₃- | CH₂ | 8-carboxamido | |
| 536 | Me | 2-Pyrazinyl- | S-(CH₂)₃- | O | H | |
| 537 | Me | 2-Thienyl | S-(CH₂)8- | O | 7-methoxy | |
| 538 | Me | 3-Jod-phenyl | O-(CH₂)₃- | CH₂ | H | |
| 539 | Me | Phenyl | S-(CH₂)₃- | O | H | |
| 540 | Hexyl | Pyridin-4-yl- | S-(CH₂)₃- | CH₂ | 7-methoxy | |
| 541 | Me | 2-Thienyl | (CH₂)₄- | CH₂ | H | |
| 542 | cycPropyl | Pyridin-4-yl- | S-(CH₂)₃- | CH₂ | 9-methyl | |
| 543 | cycPropyl | Amino | S-(CH₂)₃- | CH₂ | 9-methyl | |
| 544 | Me | 3-Jod-phenyl | S-(CH₂)₃- | O | 8-tert-butyl | |
| 545 | Ethyl | 4-Methylthiazol-5-yl | S-CH₂-C(CH₃)=CH-CH₂- | CH₂ | 8-tert-butyl | |
| 546 | Me | 3-Pyrrolyl | S-(CH₂)₃- | CH₂ | H | |
| 547 | Propyl | Phenyl | S-(CH₂)₃- | O | 8-carboxamido | |
| 548 | Me | 4-Imidazolyl- | S-(CH₂)₃- | CH₂ | 8-tert-butyl | |
| 549 | Propyl | Pyridin-3-yl- | CONH-(CH₂)₄- | CH₂ | 8-methyl | |
| 550 | isoPropyl | 4-Methoxyphenyl | S-(CH₂)₃- | O | 8-sulfonamido | |
| 551 | Me | 2-Me-4-Oxazolyl- | S-(CH₂)₃- | CH₂ | H | |
| 552 | Me | 3-Jod-phenyl | S-(CH₂)₃- | O | 8-cyano | |
| 553 | Me | 2-Thienyl | S-(CH₂)₃- | O | H | |
| 554 | cycPropyl | 3-Benzthienyl- | S-(CH₂)₃- | CH₂ | 9-methyl | |
| 555 | Me | 4-Methylthiazol-5-yl | S-(CH₂)₃- | CH₂ | 9-methyl | |
| 556 | Hexyl | Amino | S-(CH₂)₃- | CH₂ | 7-methoxy | |
| 557 | isoButyl | Tetrazolyl- | S-(CH₂)₃- | O | H | |
| 558 | Et | 4-Methoxyphenyl | S-(CH₂)₃- | CH₂ | H | |
| 559 | cycPropyl | 4-Imidazolyl- | S-(CH₂)₃- | CH₂ | 9-methyl | |
| 560 | Me | Phenyl | S-(CH₂)₃- | CH₂ | 8-cyano | |
| 561 | Me | 2-Thienyl | O-(CH₂)₄- | CH₂ | 8-tert-butyl | |
| 562 | Me | 2-Aminothiazol-4yl- | S-(CH₂)₃- | CH₂ | 9-methyl | |
| 563 | Butyl | Cyano | S-(CH₂)₃- | CH₂ | 8-methoxy | 9-methoxy |
| 564 | Me | 5-Methyl imidazol-4-yl- | S-(CH₂)₃- | CH₂ | 9-methyl | |
| 565 | Me | Phenyl | S-(CH₂)₃- | CH₂ | 7-methoxy | |
| 566 | Me | 2-Pyrazinyl- | S-(CH₂)3- | CH₂ | 8-nitro | |
| 567 | Phenyl | 3-Benzthienyl- | S-(CH₂)3- | CH₂ | H | |
| 568 | Me | 2,5-Di-methyl-furanyl-3- | S-(CH₂)₃- | O | H | |
| 569 | Et | Pyridin-4-yl- | S-(CH₂)₃- | CH₂ | H | |
| 570 | Me | Tetrazolyl- | S-(CH₂)₃- | CH₂ | 8-tert-butyl | |
| 571 | Me | 2-Thienyl | S-(CH₂)3- | CH₂ | 8-cyano | |
| 572 | Me | 4-Methylthiazol-5-yl | S-(CH₂)3- | CH₂ | 8-cyano | |
| 573 | Me | N-Methyl-2-Pyrrolyl- | S-(CH₂)₃- | CH₂ | 8-sulfonamido | |
| 574 | Hexyl | 4-Methoxyphenyl | S-(CH₂)₃- | CH₂ | 7-methoxy | |
| 575 | Me | 2-Pyrazinyl- | S-(CH₂)3- | O | 8-nitro | |
| 576 | Et | N-Propyl-tetrazolyl- | S-(CH₂)3- | CH₂ | H | |
| 577 | Me | 3-Pyrrolyl | S-(CH₂)₃- | CH₂ | 8-tert-butyl | |
| 578 | Me | 2-Pyrazinyl- | S-(CH₂)₃- | CH₂ | 7-methoxy | |
| 579 | Me | 4-Methoxyphenyl | (CH₂)₄- | O | 10-fluor | |
| 580 | Ethyl | Pyridin-3-yl- | S-CH₂-CH=CH-CH₂- | CH₂ | 9-fluor | |
| 581 | Ethyl | N-Methyl-2-Pyrrolyl- | (CH₂)₄- | O | 10-methyl | |
| 582 | Me | 5-Methyl imidazol-4-yl- | S-(CH₂)₃- | O | H | |
| 583 | Ethyl | 4-Methylthiazol-5-yl | S-CH₂-C(=CH₂)-CH₂ | O | 8-sulfonamido | |
| 584 | Me | 2,5-Di-methyl-furanyl-3- | S-(CH₂)3- | CH₂ | 8-tert-butyl | H |
| 585 | Me | 3-Jod-phenyl | S-(CH₂)₃- | CH₂ | 8-nitro | |
| 586 | Me | N-Methyl-2-Pyrrolyl- | S-(CH₂)₃- | O | H | |
| 587 | Me | 2-Thienyl | S-(CH₂)₃- | CH₂ | 8-nitro | |
| 588 | cycPropyl | Methylamino | S-(CH₂)3- | CH₂ | H | |
| 589 | Me | 4-Methylthiazol-5-yl | S-(CH₂)3- | CH₂ | 7-methoxy | |
| 590 | Ethyl | 4-Methylthiazol-5-yl | S-CH₂-C(CH₃)=CH-CH₂- | | 8-tert-butyl | |
| 591 | Propyl | 3-Jod-phenyl | COO-(CH₂)₄- | CH₂ | 9-trifluormethoxy | |

| | | | | | | |
|---|---|---|---|---|---|---|
| * Soweit für R⁷ keine Bedeutung angegeben ist, steht R⁷ für Wasserstoff. | | | | | | |

In prinzipiell analoger Weise können hergestellt werden:

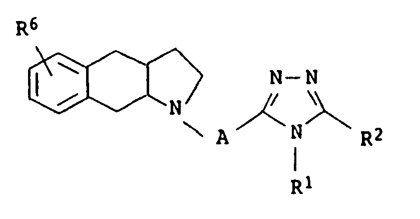

**Tabelle 3:**

| Bsp | R¹ | R² | A | R⁶ |
|---|---|---|---|---|
| 592 | Me | Tetrazolyl- | S-(CH₂)₃- | 7-tert-butyl |
| 593 | Me | 3-Jod-phenyl | O-(CH₂)₃- | 7-nitro |
| 594 | Me | 4-Methoxyphenyl | S-CH₂-CH=CH-CH₂- | 6-methoxy |
| 595 | Me | Amino | S-CH₂-C(CH₃)=CH-CH₂- | 7-tert-butyl |
| 596 | Me | Methylamino | S-(CH₂)₃- | 7-tert-butyl |
| 597 | Propyl | 4-Methoxyphenyl | S-(CH₂)₃- | 7-nitro |
| 598 | Me | 3-Pyrrolyl | S-(CH₂)₃- | 7-tert-butyl |
| 599 | Me | 3-Jod-phenyl | S-(CH₂)₄- | 6-methoxy |
| 600 | Me | 2-Pyrazinyl- | S-(CH₂)₃- | 7-nitro |
| 601 | isoPropyl | 2-Thienyl | CONH-(CH₂)₄- | H |
| 602 | Me | 4-Methylthiazol-5-yl | S-(CH₂)₃- | 6-chloro |
| 603 | Me | 2-Pyrazinyl- | S-(CH₂)₃- | 6-chloro |
| 604 | Butyl | Oxadiazol-2-yl | S-(CH₂)₃- | 7-tert-butyl |
| 605 | cycPropyl | Phenyl | (CH₂)₄- | 6-methyl |
| 606 | Me | 4-Imidazolyl- | S-(CH₂)₃- | 7-tert-butyl |
| 607 | Phenyl | 3-Benzthienyl- | S-(CH₂)₃- | 7-tert-butyl |
| 608 | Propyl | 2-Thienyl | S-CH₂-CH=CH-CH₂- | 6-methoxy |
| 609 | Hexyl | Phenyl | (CH₂)₄- | 6-methyl |
| 610 | Propyl | Phenyl | S-CH₂-C(CH₃)=CH-CH₂- | 6-methyl |
| 611 | isoPropyl | Phenyl | S-CH₂-C(=CH₂)-CH₂ | 6-methyl |
| 612 | Me | N-Methyl-2-Pyrrolyl- | S-(CH₂)₃- | 6-methoxy |
| 613 | Me | 4-Jod-phenyl | S-CH₂-CH=CH-CH₂- | 7-tert-butyl |
| 614 | Me | Pyridin-3-yl- | S-CH₂-CH=CH-CH₂- | 7-tert-butyl |
| 615 | Propyl | Phenyl | S-(CH₂)₃- | 6-methoxy |
| 616 | Me | 3-Jod-phenyl | S-(CH₂)₃- | 6-chloro |
| 617 | Me | 3-Thienyl | S-(CH₂)₃- | 7-tert-butyl |
| 618 | Ethyl | Phenyl | (CH₂)₈- | H |
| 619 | Me | Cyclohexyl- | S-(CH₂)₃- | 7-tert-butyl |
| 620 | Me | Pyridin-3-yl- | S-(CH₂)₃ | H |
| 621 | Me | Phenyl | S-(CH₂)₃- | 7-cyano |
| 622 | Ethyl | Phenyl | S-(CH₂)₃- | 6-methyl |
| 623 | Me | Pyridin-4-yl- | O-(CH₂)₃- | 7-tert-butyl |
| 624 | Ethyl | 4-Methylthiazol-5-yl | S-CH₂-C(CH₃)=CH-CH₂- | 7-cyano |
| 625 | Me | 2-Pyrazinyl- | S-(CH₂)₃- | 6-methoxy |
| 626 | Ethyl | Phenyl | S-(CH₂)₃- | 6-chloro |
| 627 | Ethyl | 3-Jod-phenyl | S-CH₂-C(CH₃)==CH-CH₂- | 7-cyano |
| 628 | Me | 2-Thienyl | S-(CH₂)₃- | 7-nitro |
| 629 | Ethyl | Pyridin-3-yl- | S-(CH₂)₃- | 7-cyano |
| 630 | Me | N-Methyl-2-Pyrrolyl- | S-(CH₂)₃- | 6-chloro |
| 631 | Me | N-Methyl-2-Pyrrolyl- | S-(CH₂)₃- | 7-tert-butyl |
| 632 | isoPropyl | 4-Methoxyphenyl | S-(CH₂)₃- | 7-tert-butyl |
| 633 | Me | 3-Br-Pyridin-5-yl- | S-CH₂-C(CH₃)=CH-CH₂- | 7-tert-butyl |
| 634 | isoPropyl | 3-Cyano-phenyl | O-(CH₂)₈- | 7-tert-butyl |
| 635 | Ethyl | Phenyl | S-(CH₂)₃- | 7-nitro |
| 636 | Me | 5-Methyl imidazol-4-yl- | S-(CH₂)₃- | 7-tert-butyl |
| 637 | Ethyl | N-Methyl-2-Pyrrolyl- | S-(CH₂)₃- | 7-cyano |
| 638 | Me | Pyridin-3-yl- | S-(CH₂)₃- | 6-chloro |
| 639 | Me | 4-Methylthiazol-5-yl | S-(CH₂)₃- | 6-methoxy |
| 640 | Phenyl | 2-Pyrazinyl- | S-CH₂-C(CH₃)=CH-CH₂- | 7-tert-butyl |
| 641 | Hexyl | Phenyl | (CH₂)₄- | 6-methyl |
| 642 | Me | 4-Methoxyphenyl | S-(CH₂)₃- | 6-chloro |
| 643 | Me | N-Methyl-2-Pyrrolyl- | S-(CH₂)₃- | 7-nitro |
| 644 | Me | 2,5-Di-methyl-furanyl-3- | S-(CH₂)₃- | 7-tert-butyl |
| 645 | Propyl | Phenyl | COO-(CH₂)₄- | H |
| 646 | cycPropyl | Phenyl | O-(CH₂)₃- | 6-methyl |
| 647 | Me | 2-Thienyl | S-(CH₂)₇- | 6-chlor |
| 648 | isoPropyl | 2-Aminothiazol-4yl- | S-(CH₂)₃- | 7-tert-butyl |
| 649 | Butyl | Phenyl | (CH₂)₄- | 6-methyl |
| 650 | Me | 2-Pyrazinyl- | S-(CH₂)₃- | 7-cyano |
| 651 | Me | 4-Methoxyphenyl | S-CH₂-CH=CH-CH2- | H |
| 652 | Me | N-Propyl-tetrazolyl- | S-(CH₂)₃- | 7-tert-butyl |
| 653 | Me | Pyridin-3-yl- | S-(CH₂)₃- | 6-methoxy |
| 654 | Butyl | Phenyl | CO-(CH₂)₃- | 6-methyl |
| 655 | Me | 2-Me-4-Oxazolyl- | S-CH₂-CH=CH-CH₂- | 7-tert-butyl |
| 656 | Me | 4-Methylthiazol-5-yl | S-(CH₂)₉- | 7-nitro |
| 657 | Me | 2-Thienyl | S-(CH₂)₃- | 7-cyano |

In prinzipiell analoger Weise können hergestellt werden:

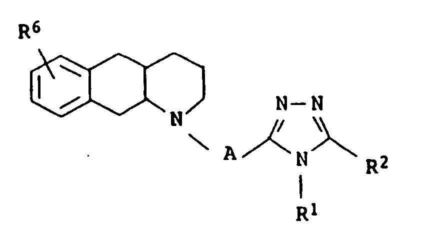

**Tabelle 4:**

| Bsp | R¹ | R² | A | R⁶ |
|---|---|---|---|---|
| 658 | Me | N-Methyl-2-Pyrrolyl- | S-(CH₂)₃- | 7-methoxy |
| 659 | Me | 2-Pyrazinyl- | S-(CH₂)₃- | 7-methoxy |
| 660 | Me | Amino | S-CH₂-C(CH₃)=CH-CH₂- | 8-tert-butyl |
| 661 | Me | 4-Methylthiazol-5-yl | S-(CH₂)₉- | 8-nitro |
| 662 | Me | 4-Imidazolyl- | S-(CH₂)₃- | 8-tert-butyl |
| 663 | Ethyl | Pyridin-3-yl- | S-(CH₂)₃- | 8-cyano |
| 664 | Me | Pyridin-3-yl- | S-(CH₂)₃- | 7-chlor |
| 665 | Me | N-Methyl-2-Pyrrolyl- | S-(CH₂)₃- | H |
| 666 | Ethyl | Phenyl | (CH₂)₈- | H |
| 667 | Me | 3-Jod-phenyl | O-(CH₂)₃- | 8-nitro |
| 668 | Ethyl | Phenyl | S-(CH₂)₃- | 7-methyl |
| 669 | Me | Pyridin-4-yl- | S-CH₂-C(CH₃)=CH-CH₂- | 8-tert-butyl |
| 670 | Me | 4-Methoxyphenyl | S-CH₂-CH=CH-CH₂- | 7-methoxy |
| 671 | Me | Pyridin-3-yl- | S-CH₂-CH=CH-CH₂- | 8-tert-butyl |
| 672 | Propyl | 4-Methoxyphenyl | S-(CH₂)₃- | 8-nitro |
| 673 | Me | 3-Pyrrolyl | S-(CH₂)₃- | 8-tert-butyl |
| 674 | Propyl | 2-Thienyl | S-CH₂-CH=CH-CH₂- | 7-methoxy |
| 675 | Me | N-Methyl-2-Pyrrolyl- | S-(CH₂)₃- | 8-nitro |
| 676 | Butyl | Phenyl | CO-(CH₂)₃- | 7-methyl |
| 677 | Me | 2-Aminothiazol-4yl- | S-(CH₂)₃- | 8-tert-butyl |
| 678 | Me | Pyridin-3-yl- | S-(CH₂)₃- | H |
| 679 | Ethyl | N-Methyl-2-Pyrrolyl- | S-(CH₂)₃- | 8-cyano |
| 680 | Me | 2-Thienyl | S-(CH₂)₃- | 8-cyano |
| 681 | isoPropyl | 5-Methyl imidazol-4-yl- | S-(CH₂)₃- | 8-tert-butyl |
| 682 | Butyl | Oxadiazol-2-yl | S-CH₂-C(CH₃)=CH-CH₂- | 8-tert-butyl |
| 683 | Me | 4-Methylthiazol-5-yl | S-(CH₂)₃- | H |
| 684 | Hexyl | Phenyl | (CH₂)₄- | 7-methyl |
| 685 | Me | 3-Br-Pyridin-5-yl- | S-CH₂-C(CH₃)=CH-CH₂- | 8-tert-butyl |
| 686 | Propyl | Phenyl | S-CH₂-C(CH₃)=CH-CH₂- | 7-methyl |
| 687 | Me | 2-Pyrazinyl- | S-(CH₂)₃- | 8-nitro |
| 688 | Me | 2-Thienyl | S-(CH₂)₃- | 8-nitro |
| 689 | Me | Phenyl | S-(CH₂)₃- | 8-cyano |
| 690 | Me | 3-Jod-phenyl | S-(CH₂)₄- | 7-methoxy |
| 691 | Me | Tetrazolyl- | S-(CH₂)₃- | 8-tert-butyl |
| 692 | Me | 2-Pyrazinyl- | S-(CH₂)₃- | H |
| 693 | Me | 2-Pyrazinyl- | S-(CH₂)₃- | 8-cyano |
| 694 | Me | 2,5-Di-methyl-furanyl-3- | S-(CH₂)₃- | 8-tert-butyl |
| 695 | Me | 4-Methylthiazol-5-yl | S-(CH₂)₃- | 7-methoxy |
| 696 | Phenyl | 3-Benzthienyl- | S-(CH₂)₃- | 8-tert-butyl |
| 697 | Propyl | Phenyl | COO-(CH₂)₄- | H |
| 698 | isoPropyl | Phenyl | S-CH₂-C(=CH₂)-CH₂ | 7-methyl |
| 699 | Ethyl | 3-Jod-phenyl | S-CH₂-C(CH₃)=CH-CH₂- | 8-cyano |
| 700 | Phenyl | 2-Pyrazinyl- | O-(CH₂)₃- | 8-tert-butyl |
| 701 | Me | 2-Me-4-Oxazolyl- | S-CH₂-CH=CH-CH₂- | 8-tert-butyl |
| 702 | isoPropyl | Cyclohexyl- | O-(CH₂)₈- | 8-tert-butyl |
| 703 | Phenyl | 3-Cyano-phenyl | S-(CH₂)₃- | 8-tert-butyl |
| 704 | Me | Pyridin-3-yl- | S-(CH₂)₃- | 7-methoxy |
| 705 | Me | 4-Methoxyphenyl | S-(CH₂)₃- | 8-tert-butyl |
| 706 | Me | N-Propyl-tetrazolyl- | S-(CH₂)₃- | 8-tert-butyl |
| 707 | Me | 4-Methoxyphenyl | S-(CH₂)₃- | 7-chlor |
| 708 | Propyl | Phenyl | S-(CH₂)₃- | 7-methoxy |
| 709 | Me | 2-Thienyl | S-(CH₂)₇- | H |
| 710 | cycPropyl | Phenyl | (CH₂)₄- | 7-methyl |
| 711 | Ethyl | Phenyl | S-(CH₂)₃- | 8-nitro |
| 712 | cycPropyl | Phenyl | O-(CH₂)₃- | 7-methyl |
| 713 | Me | 4-Jod-phenyl | S-CH₂-CH=CH-CH₂- | 8-tert-butyl |
| 714 | Ethyl | 4-Methylthiazol-5-yl | S-CH₂-C(CH₃)=CH-CH₂- | 8-cyano |
| 715 | Me | 4-Methoxyphenyl | S-CH₂-CH=CH-CH₂- | H |
| 716 | Me | Methylamino | S-(CH₂)₃- | 8-tert-butyl |
| 717 | isoPropyl | 3-Thienyl | S-(CH₂)₃- | 8-tert-butyl |
| 718 | Me | 3-Jod-phenyl | S-(CH₂)₃- | 7-chlor |
| 719 | Butyl | Phenyl | (CH₂)₄- | 7-methyl |
| 720 | Hexyl | Phenyl | (CH₂)₄- | 7-methyl |
| 721 | isoPropyl | 2-Thienyl | CONH-(CH₂)₄- | H |
| 722 | Me | N-Methyl-2-Pyrrolyl- | S-(CH₂)₃- | 8-tert-butyl |
| 723 | Ethyl | Phenyl | S-(CH₂)₃- | 7-chloro |

Beispiele für galenische Applikationsformen

### A) Tabletten

Auf einer Tablettenpresse werden in üblicher Weise Tabletten folgender Zusammensetzung gepresst:

| | |
|---|---|
| 40 mg | Substanz des Beispiels 1 |
| 120 mg | Maisstärke |
| 13,5 mg | Gelatine |
| 45 mg | Milchzucker |
| 2,25mg | Aerosil^{®} (chemisch reine Kieselsäure in submikroskopisch feiner Verteilung) |
| 6,75mg | Kartoffelstärke (als 6%-iger Kleister) |

### B) Dragees

| | |
|---|---|
| 20 mg | Substanz des Beispiels 1 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus
5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptorexprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10% fötalem Kälberserum (GIBCO Nr. 041-32400 N); 100 E/ml Penicillin und 0,2% Streptomycin (GIBCO BRL, Gaithersburg, MD, USA) vermehrt. Nach 48 h wurden die Zellen mit PBS gewaschen und mit 0,05% trypsinhaltiger PBS 5 min inkubiert. Danach wurde mit Medium neutralisiert und die Zellen durch Zentrifugation bei 300 g gesammelt. Zur Lyse der Zellen wurde kurz das Pellet mit Lysispuffer (5mM Tris-HCl, pH 7,4 mit 10% Glycerin) gewaschen und danach in einer Konzentration von 10⁷-Zellen/ml Lysispuffer 30 min bei 4°C inkubiert. Die Zellen wurden bei 200 g 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10 µm Quinolinol, 0,1% Ascorbinsäure und 0,1% BSA) in einer Konzentration von ca. 10⁶ Zellen/250 µl Testansatz suspendiert und bei 30°c mit 0,1 nM ¹²⁵Jodsulpirid in Anwesenheit und Abwesenheit von Testsubstanz inkubiert. Die unspezifische Bindung wurde mit 10⁻⁶ M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND. Für die Verbindung aus Beispiel 1 beträgt der Kᵢ-Wert für die Bindung an den D₃-Rezeptor Kᵢ = 50 nM.

### 2) D₂-Bindungstest

### Zellkultur

HEK-293 Zellen mit stabil exprimierten humanen Dopamin-D2A-Rezeptoren wurden in RPMI 1640 mit Glutamax I^{™} und 25 mM HE-PES mit 10% fötalem Kälberserumalbumin kultiviert. Alle Medien enthielten 100 Einheiten pro ml Penicillin und 100 µg/ml Streptomycin. Die Zellen wurden in feuchter Atmosphäre mit 5% CO₂ bei 37°C gehalten.

Die zellpräparation für Bindungsstudien erfolgte durch Trypsinisierung (0,05% Trypsinlösung) für 3-5 Minuten bei Raumtemperatur. Danach wurden die Zellen bei 250 g 10 Minuten zentrifugiert und 30 Minuten bei 4°C mit Lysispuffer (5 mM Tris-HCl, 10% Glycerol, pH 7,4) behandelt. Nach Zentrifugation bei 250 g für 10 Minuten wurde der Rückstand bei -20°C bis zum Gebrauch aufbewahrt.

### Rezeptorbindungstests

### Dopamin-D₂-Rezeptor "low affinity state" mit ¹²⁵I-Spiperon (81 TBq/mmol, Du Pont de Nemours, Dreieich)

Die Ansätze (1 ml) setzten sich zusammen aus 1 x 10⁵ Zellen in Inkubationspuffer (50 mM Tris, 120 mM NaCl, 5 mM KCl, 2 mM MgCl₂ und 2 mM CaCl₂, pH 7,4 mit HCl) und 0,1 nM ¹²⁵I-Spiperon (totale Bindung) oder zusätzlich 1 µM Haloperidol (unspezifische Bindung) oder Prüfsubstanz.

Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.

Die Auswertung erfolgte wie unter a).

Die Bestimmung der Kᵢ-Werte erfolgte über nichtlineare Regressionsanalyse mit dem Programm LIGAND oder durch Umrechnung der IC₅₀-Werte mit Hilfe der Formel von Cheng und Prusoff.

Die erfindungsgemäßen Verbindungen zeigen in diesen Tests sehr gute Affinitäten am D₃-Rezeptor (< 1 µmolar, insbesondere < 200 nmolar) und binden selektiv an den D₃-Rezeptor.

## Patentansprüche

1. Triazolverbindungen der allgemeinen Formel I 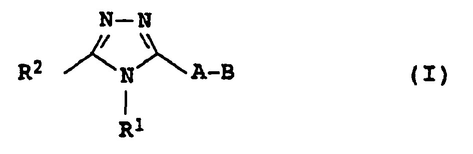 worin
R¹ für H, C₁-C₆-Alkyl, das gegebenenfalls durch OH, OC₁-C₆-Alkyl, Halogen oder Phenyl substituiert ist, C₃-C₆-Cycloalkyl oder Phenyl steht;
R² für H, C₁-C₆-Alkyl, das gegebenenfalls durch OH, OC₁-C₆-Alkyl, Halogen oder Phenyl substituiert ist, C₁-C₆-Alkoxy, C₁-C₆-Alkylthio, C₂-C₆-Alkenyl, C₂-C₆-Alkinyl, C₃-C₆-Cycloalkyl, Halogen, CN, COOR³, CONR³R⁴, NR³R⁴, SO₂R³, SO₂NR³R⁴ oder einen aromatischen Rest steht, der ausgewählt ist unter Phenyl, Naphthyl und einem 5- oder 6-gliedrigen heterocyclischen Rest mit 1, 2, 3 oder 4 Heteroatomen, die unabhängig voneinander ausgewählt sind unter 0, N und S, wobei der aromatische Rest einen oder zwei Substituenten aufweisen kann, die unabhängig voneinander ausgewählt sind unter C₁-C₆-Alkyl, das gegebenenfalls durch OH, OC₁-C₆-Alkyl, Halogen oder Phenyl substituiert ist, C₁-C₆-Alkoxy, C₂-C₆-Alkenyl, C₂-C₆-Alkinyl, C₃-C₆-Cycloalkyl, Halogen, CN, COR³, NR³R⁴, NO₂, SO₂R³, SO₂NR³R⁴ und Phenyl, das gegebenenfalls durch ein oder zwei Reste substituiert ist, die unabhängig voneinander ausgewählt sind unter C₁-C₆-Alkyl, C₁-C₆-Alkoxy, NR³R⁴, CN, CF₃, CHF₂ oder Halogen;
R³ und R⁴ unabhängig voneinander für H, C₁-C₆-Alkyl, das gegebenenfalls durch OH, OC₁-C₆-Alkyl, Halogen oder Phenyl substituiert ist, oder Phenyl, stehen;
A für C₄-C₁₀-Alkylen oder C₃-C₁₀-Alkylen, das wenigstens eine Gruppe Z umfasst, die ausgewählt ist unter O, S, CONR³, COO, CO, C₃-C₆-Cycloalkyl und einer Doppel- oder Dreifachbindung, steht;
B einen Rest der folgenden Formeln (a) oder (b) bedeutet: 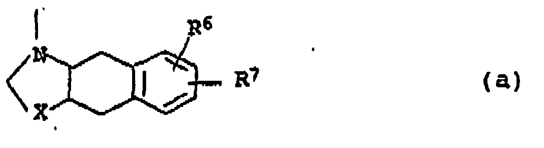 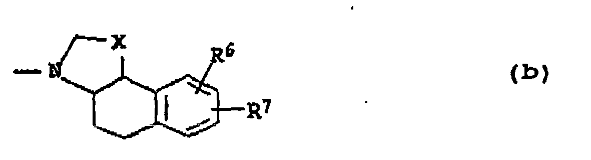
X für CH₂ oder CH₂CH₂ steht;
R⁶ und R⁷ unabhängig voneinander ausgewählt sind unter H, C₁-C₆-Alkyl, das gegebenenfalls durch Halogen substituiert ist, OH, C₁-C₆-Alkoxy, C₂-C₆-Alkenyl, Halogen, CN, NO₂, SO₂R³, SO₂NR³R⁴ und CONR³R⁴,
sowie deren Salze mit physiologisch verträglichen Säuren.

2. Verbindungen nach Anspruch 1 der Formel I, worin X für -CH₂- steht.

3. Verbindungen nach Anspruch 1 oder 2 der Formel I, worin A für C₄-C₁₀-Alkylen oder C₃-C₁₀-Alkylen, das wenigstens eine Gruppe Z umfasst, die ausgewählt ist-unter O, S, coo, Co, einer Doppel- oder Dreifachbindung und C₃-C₆-Cycloalkyl steht

4. Verbindungen nach einem der vorhergehenden Ansprüche der Formel I, worin A für C₄-C₁₀-Alkylen oder C₃-C₁₀-Alkylen steht, das wenigstens eine Gruppe Z umfasst, die ausgewählt ist unter O, S, einer Doppelbindung und Cyclohexyl.

5. Verbindungen nach einem der vorhergehenden Ansprüche der Formel I, worin R² für einen aromatischen Rest steht, der unsuristituiert ist oder einen oder zwei Substituenten aufweist, die unabhängig voneinander ausgewählt sind unter C₁-C₆-Alkyl, OH, C₁-C₆-Alkoxy, Phenyl, CN und Halogen.

6. verbindungen nach einem der vorhergehenden Ansprüche der Formel I, worin R² für H, C₁-C₆-Alkyl, Phenyl, Thienyl, Furanyl, Pyridyl, Pyrrolyl, Thiazolyl oder Pyrazinyl steht.

7. Verbindungen nach einem der vorhergehenden Ansprüche der Formel I, worin R¹ für H, C₁-C₆-Alkyl oder C₃-C₆-Cycloalkyl steht.

8. Verbindungen nach einem der vorhergehenden Ansprüche der Formel I, worin R⁶ und R⁷ unabhängig voneinander ausgewählt sind unter H, C₁-C₆-Alkyl, OH, C₁-C₆-Alkoxy, C₁-C₆-Alkylthio-C₁-C₆-alkyl, Halogen, CN, NO₂, SO₂R³, SO₂NR³R⁴, und CONR³R⁴.

9. Verbindungen nach Anspruch 1 der Formel I, worin
R¹ für H, C₁C₆-Alkyl oder Phenyl steht,
R² für H, C₁-C₆-Alkyl, Phenyl, Thienyl, Furanyl, Tetrazolyl, Pyrrolyl, Thiazolyl oder Pyrazinyl steht,
A für -SC₃-C₁₀-Alkylen, das gegebenenfalls eine Doppelbindung umfassen kann, steht, und
R⁶ und R⁷ ausgewählt sind unter H, C₁-C₆-Alkyl, C₁-C₆-Alkoxy, Halogen, SO₂NR³R⁴, CN, NO₂ und CF₃.

10. Pharmazeutisches Mittel, enthaltend wenigstens eine Verbindung nach einem der Ansprüche 1 bis 9, gegebenenfalls zusammen mit physiologisch akzeptablen Trägern und/oder Hilfsstoffen.

11. Verwendung wenigstens einer Verbindung nach einem der Ansprüche 1 bis 9 zur Herstellung eines pharmazeutischen Mittels zur Behandlung von Erkrankungen, die auf die Beeinflussung durch Dopamin-D₃-Rezeptorantagonisten bzw. -agonisten ansprechen.

## Claims

1. A triazole compound of the formula I 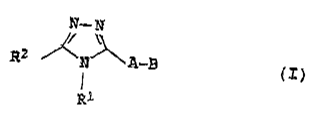 where
R¹ is H, C₁-C₆-alkyl, which is optionally substituted by OH, OC₁-C₆-alkyl, halogen or phenyl, C₃-C₆-cycloalkyl or phenyl;
R² is H, C₁-C₆-alkyl, which is optionally substituted by OH, OC₁-C₆-alkyl, halogen or phenyl, C₁-C₆-alkoxy, C₁-C₆-alkylthio, C₂-C₆-alkenyl, C₂-C₆-alkynyl, C₃-C₆-cycloalkyl, halogen, CN, COOR³, CONR³R⁴, NR³R⁴, SO₂R³, SO₂NR³R⁴ or an aromatic radical which is selected from phenyl, naphthyl and a 5- or 6-membered heterocyclic radical having 1, 2, 3 or 4 heteroatoms which are selected, independently of each other, from O, N and S, with it being possible for the aromatic radical to have one or two substituents which are selected, independently of each other, from C₁-C₆-alkyl, which is optionally substituted by OH, OC₁-C₆-alkyl, halogen or phenyl, C₁-C₆-alkoxy, C₂-C₆-alkenyl, C₂-C₆-alkynyl, C₃-C₆-cycloalkyl, halogen, CN, COR³, NR³R⁴, NO₂, SO₂R³, SO₂NR³R⁴ and phenyl which is optionally substituted by one or two radicals which are selected, independently of each other, from C₁-C₆-alkyl, C₁-C₆-alkoxy, NR³R⁴, CN, CF₃, CHF₂ or halogen;
R³ and R⁴ are, independently of each other, H, C₁-C₆-alkyl, which is optionally substituted by OH, OC₁-C₆-alkyl, halogen or phenyl, or phenyl;
A is C₄-C₁₀-alkylene or C₃-C₁₀-alkylene which comprises at least one group Z which is selected from O, S, CONR³, COO, CO, C₃-C₆-cycloalkyl and a double or triple bond;
B is a radical of the following formulae (a) or (b) : 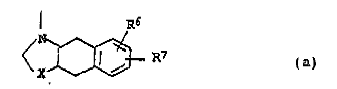 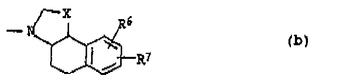
X is CH₂ or CH₂CH₂;
R⁶ and R⁷ are, independently of each other, selected from H, C₁-C₆-alkyl, which is optionally substituted by halogen, OH, C₁-C₆-alkoxy, C₂-C₆-alkenyl, halogen, CN, NO₂, SO₂R³, SO₂NR³R⁴ and CONR³R⁴,
and the salts thereof with physiologically tolerated acids.

2. The compound according to claim 1 of the formula I, where X is -CH₂-.

3. The compound according to claim 1 or 2 of the formula I, where A is C₄-C₁₀-alkylene or C₃-C₁₀-alkylene which comprises at least one group Z which is selected from O, S, COO, CO, a double bond or triple bond and C₃-C₆-cycloalkyl.

4. The compound according to one of the preceding claims of the formula I, where A is C₄-C₁₀-alkylene of C₃-C₁₀-alkylene which comprises at least one group Z which is selected from O, S, a double bond and cyclohexyl.

5. The compound according to one of the preceding claims of the formula I, where R² is an aromatic radical which is unsubstituted or has one or two substituents which are selected, independently of each other, from C₁-C₆-alkyl, OH, C₁-C₆-alkoxy, phenyl, CN and halogen.

6. The compound according to one of the preceding claims of the formula I, where R² is H, C₁-C₆-alkyl, phenyl, thienyl, furanyl, pyridyl, pyrrolyl, thiazolyl or pyrazinyl.

7. The compound according to one of the preceding claims of the formula I, where R¹ is H, C₁-C₆-alkyl or C₃-C₆-cycloalkyl.

8. The compound according to one of the preceding claims of the formula I, where R⁶ and R⁷ are selected, independently of each other, from H, C₁-C₆-alkyl, OH, C₁-C₆-alkoxy, C₁-C₆-alkylthio-C₁-C₆-alkyl, halogen, CN, NO₂, SO₂R³, SO₂NR³R⁴ and CONR³R⁴.

9. The compound according to claim 1 of the formula I, where
R¹ is H, C₁-C₆-alkyl or phenyl,
R² is H, C₁-C₆-alkyl, phenyl, thienyl, furanyl, tetrazolyl, pyrrolyl, thiazolyl or pyrazinyl,
A is -SC₃-C₁₀-alkylene which may, if appropriate, comprise a double bond, and
R⁶ and R⁷ are selected from H, C₁-C₆-alkyl, C₁-C₆-alkoxy, halogen, SO₂NR³R⁴, CN, NO₂ and CF₃.

10. A pharmaceutical which comprises at least one compound according to one of claims 1 to 9, if appropriate together with physiologically acceptable excipients and/or adjuvants.

11. The use of at least one compound according to one of claims 1 to 9 in the manufacture of a pharmaceutical for treating diseases which respond to the influence of dopamine D₃ receptor antagonists or agonists.

## Revendications

1. Composés de triazole de la formule générale I 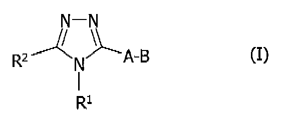 dans laquelle
R¹ représente H, un groupe alkyle en C₁-C₆, lequel est éventuellement substitué par OH, O(alkyle en C₁-C₆), halogène ou phényle ; un groupe cycloalkyle en C₃-C₆ ou phényle ;
R² représente H, alkyle en C₁-C₆ qui est éventuellement substitué par OH, O(alkyle en C₁-C₆), halogène ou phényle ; un groupe alcoxy en C₁-C₆, (alkyle en C₁-C₆)thio, alcényle en C₂-C₆, alcynyle en C₂-C₆, cycloalkyle en C₃-C₆, un atome d'halogène, CN, COOR³, CONR³R⁴, NR³R⁴, SO₂R³, SO₂NR³R⁴ ou un reste aromatique, lequel est choisi parmi un groupe phényle, naphtyle et un reste hétérocyclique à 5 ou 6 éléments avec 1, 2, 3 ou 4 hétéroatomes qui sont choisis indépendamment parmi O, N et S, le reste aromatique pouvant présenter un ou deux substituants qui sont choisis indépendamment parmi un groupe alkyle en C₁-C₆, lequel est éventuellement substitué par OH, un groupe O(alkyle en C₁-C₆), un atome d'halogène ou un groupe phényle ; un groupe alcoxy en C₁-C₆, alcényle en C₂-C₆, alcynyle en C₂-C₆, cycloalkyle en C₃-C₆, un atome d'halogène, CN, COR³, NR³R⁴, NO₂, SO₂R³, SO₂NR³R⁴ et un groupe phényle, lequel est éventuellement substitué par un ou deux restes qui sont choisis indépendamment parmi un groupe alkyle en C₁-C₆, alcoxy en C₁-C₆, NR³R⁴, CN, CF₃, CHF₂ ou un atome d'halogène ;
R³ et R⁴ représentent indépendamment H, un groupe alkyle en C₁-C₆ qui est éventuellement substitué par OH, O(alkyle en C₁-C₆), halogène ou phényle ;
A représente un groupe alkylène en C₄-C₁₀ ou alkylène en C₃-C₁₀, lequel comprend au moins un groupe Z qui est choisi parmi O, S, CONR³, COO, CO, un groupe cycloalkyle en C₃-C₆ et une double ou triple liaison ;
B représente un reste des formules (a) ou (b) suivantes : 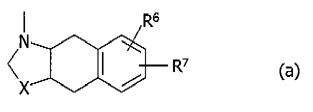 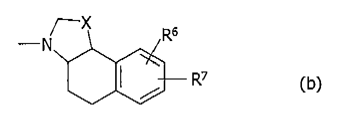
X représente CH₂ ou CH₂CH₂ ;
R⁶ et R⁷ sont choisis indépendamment parmi H, un groupe alkyle en C₁-C₆ qui est éventuellement substitué par halogène, OH, un groupe alcoxy en C₁-C₆, alcényle en C₂-C₆, un atome d'halogène, CN, NO₂, SO₂R³, SO₂NR³R⁴ et CONR³R⁴,
ainsi que des sels de ceux-ci avec des acides physiologiquement compatibles.

2. Composés selon la revendication 1 de la formule I, dans laquelle X représente -CH₂-.

3. Composés selon la revendication 1 ou 2 de la formule I, dans laquelle A représente un groupe alkylène en C₄-C₁₀ ou alkylène en C₃-C₁₀ qui comprend au moins un groupe Z qui est choisi parmi O, S, COO, CO, une double ou triple liaison et un groupe cycloalkyle en C₃-C₆.

4. Composés selon l'une quelconque des revendications précédentes de la formule I, dans laquelle A représente un groupe alkylène en C₄-C₁₀ ou alkylène en C₃-C₁₀, lequel comprend au moins un groupe Z qui est choisi parmi O, S, une double liaison et un groupe cyclohexyle.

5. Composés selon l'une quelconque des revendications précédentes de la formule I, dans laquelle R² représente un reste aromatique qui est non substitué ou qui présente un ou deux substituants qui sont choisis indépendamment parmi un groupe alkyle en C₁-C₆, OH, un groupe alcoxy en C₁-C₆, phényle, CN et un atome d'halogène.

6. Composés selon l'une quelconque des revendications précédentes de la formule I, dans laquelle R² représente H, un groupe alkyle en C₁-C₆, phényle, thiényle, furanyle, pyridyle, pyrrolyle, thiazolyle ou pyrazinyle.

7. Composés selon l'une quelconque des revendications précédentes de la formule I, dans laquelle R¹ représente H, un groupe alkyle en C₁-C₆ ou cycloalkyle en C₃-C₆.

8. Composés selon l'une quelconque des revendications précédentes de la formule I, dans laquelle R⁶ et R⁷ sont choisis indépendamment parmi H, un groupe alkyle en C₁-C₆, OH, un groupe alcoxy en C₁-C₆, un groupe (alkyle en C₁-C₆)thio(alkyle en C₁-C₆), un atome d'halogène, CN, NO₂, SO₂R³, SO₂NR³R⁴ et CONR³R⁴.

9. Composés selon la revendication 1 de la formule I, dans laquelle
R¹ représente H, un groupe alkyle en C₁-C₆ ou phényle,
R² représente H, un groupe alkyle en C₁-C₆, phényle, thiényle, furanyle, tétrazolyle, pyrrolyle, thiazolyle ou pyrazinyle,
A représente un groupe -S(alkylène en C₃-C₁₀), lequel peut éventuellement comprendre une double liaison, et
R⁶ et R⁷ sont choisis parmi H, un groupe alkyle en C₁-C₆, alcoxy en C₁-C₆, un atome d'halogène, SO₂NR³R⁴, CN, NO₂ et CF₃.

10. Agent pharmaceutique contenant au moins un composé selon l'une quelconque des revendications 1 à 9, éventuellement avec des véhiculeurs et/ou des auxiliaires physiologiquement compatibles.

11. Utilisation d'au moins un composé selon l'une quelconque des revendications 1 à 9 pour la préparation d'un agent pharmaceutique destiné au traitement de maladies qui sont sensibles à l'influence d'antagonistes respectivement d'agonistes de récepteurs dopaminergiques-D₃.
